(19) Europäisches Patentamt
European Patent Office
Office européen des brevets

(11) EP 4 153 557 B1

(12) **EUROPEAN PATENT SPECIFICATION**

(45) Date of publication and mention of the grant of the patent:
**03.07.2024 Bulletin 2024/27**

(21) Application number: **21723766.8**

(22) Date of filing: **11.05.2021**

(51) International Patent Classification (IPC):
***C07C 53/126*** *(2006.01)* ***C07C 51/14*** *(2006.01)*
***C12P 7/6409*** *(2022.01)* ***C07C 51/48*** *(2006.01)*
***C07C 45/48*** *(2006.01)* ***C07C 49/04*** *(2006.01)*
***C07C 29/145*** *(2006.01)* ***C07C 31/125*** *(2006.01)*
***C07C 1/24*** *(2006.01)* ***C07C 11/02*** *(2006.01)*

(52) Cooperative Patent Classification (CPC):
(C-Sets available)
**C12P 7/6409; C07C 1/24; C07C 29/145;**
**C07C 45/48; C07C 51/14; C07C 51/48** (Cont.)

(86) International application number:
**PCT/EP2021/062450**

(87) International publication number:
**WO 2021/233732 (25.11.2021 Gazette 2021/47)**

(54) **METHOD FOR PRODUCING HIGHER LINEAR FATTY ACIDS OR ESTERS**

VERFAHREN ZUR HERSTELLUNG VON HÖHEREN LINEAREN FETTSÄUREN ODER ESTERN

PROCÉDÉ DE PRODUCTION D'ESTERS OU D'ACIDES GRAS SUPÉRIEURS LINÉAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.05.2020 EP 20175376**

(43) Date of publication of application:
**29.03.2023 Bulletin 2023/13**

(73) Proprietor: **Evonik Operations GmbH**
**45128 Essen (DE)**

(72) Inventors:
- **HAAS, Thomas**
**48161 Münster (DE)**
- **RICHTER, Christian**
**48151 Münster (DE)**
- **BELLER, Matthias**
**18211 Ostseebad Nienhagen (DE)**
- **RAZZAQ, Rauf**
**Multan (PK)**
- **JACKSTELL, Ralf**
**18106 Rostock (DE)**
- **BELITZ, Florian**
**44892 Bochum (DE)**
- **GÖBEL, Jonas**
**44801 Bochum (DE)**
- **GOOSSEN, Prof., Lukas**
**45529 Hattingen (DE)**

(74) Representative: **Evonik Patent Association**
**c/o Evonik Industries AG**
**IP Management**
**Bau 1042A/PB 15**
**Paul-Baumann-Straße 1**
**45772 Marl (DE)**

(56) References cited:
**EP-A1- 3 538 506** **WO-A1-2019/158683**
**US-A- 4 158 668**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 1/24, C07C 11/02;**
**C07C 29/145, C07C 31/125;**
**C07C 45/48, C07C 49/04;**
**C07C 51/14, C07C 53/126;**
**C07C 51/48, C07C 53/126**

## Description

### FIELD OF THE INVENTION

The present invention relates to a method of producing higher linear fatty acids or esters of higher linear fatty acids using a combined biotechnological and chemical method. In particular, the present invention relates to producing linear fatty acids comprising 7 to 28 carbon atoms or esters thereof, preferably dodecanoic acid (i.e. lauric acid), via higher alkanones, preferably 6-undecanone.

### BACKGROUND OF THE INVENTION

Most of the commercial linear fatty acids are derived from oils or fats, the corresponding triglycerides. Nevertheless, these triglycerides are usually mixtures of fatty acids of different chain length. In case the need of a specific fatty acid is rising because of its specific properties, at present it cannot be produced selectively but can only be isolated from a mixture. For instance, there is a need for dodecanoic acid because of its unique surfactant property. To satisfy this need dodecanoic acid is manufactured from palm kernel or coconut oil, although these oils contain many other unwanted chain lengths fatty acids in addition. US4158668 relates to a preparation of higher linear fatty acids (e.g. C12 fatty acids) from internal olefins by hydroxy carbonylation. WO2019/158683 discloses a biotransformation of ethanol and acetate to hexanoic acid and an extraction of hexanoic acid using TOPO and an alkane. EP3538506 discloses preparation of higher internal ketones by decarboxylative ketonisation of fatty acids.

Therefore, there is a strong need for a highly selective synthesis of specific chain length fatty acid. In addition, linear fatty acids are often used in consumer products like washing powder etc. Consumers are increasingly looking for sustainable, non-petrochemical, non-tropical oil derived products. The present invention approaches these tasks. It describes a method to produce highly selective linear fatty acids from $CO_2$ and hydrogen, or CO, or CO and hydrogen, or $CO_2$, CO and hydrogen.

### FIGURES OF THE INVENTION

**Figure 1** shows the the microbial metabolic pathway for carbon-chain elongation such as (a) butyric acid ($C_4$) production by the genera *Clostridium* and *Butyrivibrio* (Kim BH, et al. Appl Environ Microbiol. 1984;48(4):764-70) and (b) hexanoic acid production postulated in *Megasphaera elsdenii* and *Clostridium kluyveri* (Khan MA. Melbourne: Victoria University; 2006).

**Figure 2** shows the overall reaction scheme according to the present invention from a short chain fatty acid to a higher linear fatty acid.

### DESCRIPTION OF THE INVENTION

The present invention attempts to solve the problem above by providing a full cycle for production of higher higher linear fatty acids or esters, that involves both a biotechnological and a chemical means.

In particular, the production of higher linear fatty acids, e.g. dodecanoic acid (i.e. lauric acid), starts from a simple substrate such as ethanol and/or acetate to produce hexanoic acid using biotechnological means. The produced hexanoic acid may then be extracted and the extracted hexanoic acid subjected to a chemical step that converts the hexanoic acid via higher alkanones, e.g. 6-undecanone, to higher linear fatty acids, e.g. lauric acid. This method has the advantage of starting from a cheap and readily available raw material for production of higher linear fatty acids, preferably lauric acid. The raw material- acetate and/or ethanol is also produced using a means that does not kill any animal or plant. Further, using the biotechnological step for producing hexanoic acid followed by the extraction step according to any aspect of the present invention results in a high and pure yield of hexanoic acid that can be then readily used for the production of higher alkanones, preferably 6-undecanone, and further to higher linear fatty acids, preferably lauric acid, using a chemical step.

In particular the above-mentioned problems are solved by a method of producing linear fatty acids comprising 7 to 28 carbon atoms or esters thereof, from ethanol and/or a linear alkanoic acid comprising 2 to 5 carbon atoms, the method comprising

(a) contacting ethanol and/or the linear alkanoic acid comprising 2 to 5 carbon atoms or any salts thereof with at least one microorganism capable of carrying out two-carbon chain elongation to produce as an intermediate a linear alkanoic acid comprising 4 to 7 carbon atoms and/or a salt thereof and/or an ester thereof, e.g. hexanoic acid, butanoic acid or pentanoic acid, and/or a salt thereof and/or an ester thereof;

(b) extracting the intermediate, its salt and/or ester thereof from (a) using at least one extractant, wherein the

extractant comprises at least one alkyl-phosphine oxide and optionally at least one alkane comprising at least 12 carbon atoms; or at least one trialkylamine and at least one alkane comprising at least 12 carbon atoms;

(c) contacting the extracted intermediate linear alkanoic acid comprising 4 to 7 carbon atoms, its salt and/or ester thereof from (b) with at least one ketonization catalyst and optionally a further alkanoic acid comprising 2 to 22 carbon atoms under suitable reaction conditions for chemical ketonization of the intermediate linear alkanoic acid comprising 4 to 7 carbon atoms, its salt and/or ester thereof to a linear alkanone comprising 7 to 28 carbon atoms, preferably 6-undecanone;

(d) contacting the linear alkanone comprising 7 to 28 carbon atoms from step (c) with at least one hydrogenation metal catalyst for catalytic hydrogenation of the linear alkanone comprising 7 to 28 carbon atoms to a corresponding secondary linear alkanol comprising 7 to 28 carbon atoms;

(e) dehydration of the secondary linear alkanol comprising 7 to 28 carbon atoms in the presence of an acidic heterogeneous catalyst to form a corresponding linear alkene comprising 7 to 28 carbon atoms;

(f) hydroxy or alkoxy carbonylation of the linear alkene comprising 7 to 28 carbon atoms to a linear fatty acid comprising 7 to 28 carbon atoms or an ester thereof in the presence of carbon monoxide, of an acid and of a catalyst comprising a transition metal.

In the method according to the present invention the linear alkanoic acid comprising 2 to 5 carbon atoms in step (a) is selected from the group consisting of acetic acid, propanoic acid, butanoic acid and pentanoic acid.

The linear alkanoic acid comprising 2 to 5 carbon atoms is preferably acetic acid and the intermediate a linear alkanoic acid comprising 4 to 7 carbon atoms in steps (a) to (c) is hexanoic acid or an ester thereof is preferably hexanoic acid or an ester thereof.

In the method of according to the present invention the linear alkanoic acid comprising 2 to 5 carbon atoms may be propanoic acid and the intermediate a linear alkanoic acid comprising 4 to 7 carbon atoms is heptanoic acid or an ester thereof.

In a preferred embodiment of the method according to the present invention the linear alkanoic acid comprising 2 to 5 carbon atoms is preferably acetic acid and the intermediate a linear alkanoic acid comprising 4 to 7 carbon atoms in steps (a) to (c) is hexanoic acid or an ester thereof, the linear alkanone comprising 7 to 28 carbon atoms steps (c) and (d) is 6-undecanone, the corresponding secondary linear alkanol comprising 7 to 28 carbon atoms in steps (d) and (e) is 6-undecanol, the linear alkene comprising 7 to 28 carbon atoms in steps (e) and (f) is 5-undecene and the linear higher fatty acid is dodecanoic acid.

The microorganism in (a) capable of carrying out carbon chain elongation to produce as an intermediate a linear alkanoic acid comprising 4 to 7 carbon atoms and/or a salt thereof and/or an ester thereof, e.g. hexanoic acid, may be any organism that may be capable of carbon-chain elongation according to Figure 1 (Jeon et al. Biotechnol Biofuels (2016) 9:129). The carbon chain elongation pathway is also disclosed in Seedorf, H., et al., 2008. The microorganisms according to any aspect of the present invention may also include microorganisms which in their wild-type form are not capable of carbon chain elongation, but have acquired this trait as a result of genetic modification. In particular, the microorganism in (a) may be selected from the group consisting of *Clostridium carboxidivorans* and *Clostridium kluyveri,* more in particular, the microorganism according to any aspect of the present invention may be *Clostridium kluyveri.*

The extraction step in (b) according to any aspect of the present invention allows for an increase in yield relative to the amount of extractants used. For example, less than 50% by weight of extractant may be used to extract the same amount of hexanoic acid as if only pure alkanes were used. Therefore, with a small volume of extractant, a larger yield of intermediate linear alkanoic acid comprising 4 to 7 carbon atoms and/or a salt thereof and/or an ester thereof, e.g. hexanoic acid, may be extracted. The extractant is also not harmful to microorganisms. Accordingly, the extractant according to any aspect of the present invention may be present when the intermediate a linear alkanoic acid comprising 4 to 7 carbon atoms , e.g. hexanoic acid, is biotechnologically produced according to any aspect of the present invention. Therefore, the aqueous medium according to any aspect of the present invention, particularly after step (b) of separating the intermediate linear alkanoic acid comprising 4 to 7 carbon atoms, e.g. hexanoic acid, may be recycled back into step (a). This step of recycling allows for the microorganisms to be recycled and reused as the extractant according to any aspect of the present invention is not toxic to the microorganisms. This step of recycling the aqueous medium in the method according to any aspect of the present invention has the further advantage of enabling the residue of the intermediate linear alkanoic acid comprising 4 to 7 carbon atoms, e.g. hexanoic acid, which was not at first instance extracted from step (b) in the first cycle, to be given a chance to be extracted a further time or as many times as the aqueous medium is recycled. Further, the intermediate linear alkanoic acid comprising 4 to 7 carbon atoms, e.g. the hexanoic acid, can be easily separated from the extractant according to any aspect of the present invention by distillation. This is because the intermediate linear alkanoic acid comprising 4 to 7 carbon atoms, e.g. acid hexanoic acid, at least distills at a significantly lower boiling point than the extractant and after the separation via distillation, the extractant may be easily recycled.

The method according to the present invention may also include a step of extracting isolated intermediate linear alkanoic acid comprising 4 to 7 carbon atoms, e.g. hexanoic acid, from an aqueous medium. An isolated intermediate linear alkanoic acid comprising 4 to 7 carbon atoms, e.g. hexanoic acid, may refer to an intermediate linear alkanoic acid comprising 4 to 7 carbon atoms, e.g. hexanoic acid, that may be separated from the medium where the intermediate linear alkanoic acid comprising 4 to 7 carbon atoms, e.g. hexanoic acid, has been produced. In one example, the intermediate linear alkanoic acid comprising 4 to 7 carbon atoms, e.g. hexanoic acid, may be produced in an aqueous medium (e.g. fermentation medium where the intermediate higher alkanoic acid, e.g. hexanoic acid, is produced by specific cells from a carbon source). The isolated intermediate linear alkanoic acid comprising 4 to 7 carbon atoms, e.g. hexanoic acid, may refer to the hexanoic acid extracted from the aqueous medium. In particular, the extracting step allows for the separation of excess water from the aqueous medium thus resulting in a formation of a mixture containing the extracted intermediate linear alkanoic acid comprising 4 to 7 carbon atoms, e.g. hexanoic acid.

The extractant may also be referred to as the ’extraction medium’ or ’extracting medium’. The extractant may be used for extracting/ isolating the intermediate linear alkanoic acid comprising 4 to 7 carbon atoms, e.g. hexanoic acid, produced according to any method of the present invention from the aqueous medium wherein the intermediate linear alkanoic acid comprising 4 to 7 carbon atoms, e.g. hexanoic acid, was originally produced. At the end of the extracted step, excess water from the aqueous medium may be removed thus resulting in the extractant containing the extracted intermediate linear alkanoic acid comprising 4 to 7 carbon atoms, e.g. hexanoic acid. In particular, at the end of the extracted step, with the intermediate linear alkanoic acid comprising 4 to 7 carbon atoms acid, e.g. hexanoic acid, extracted and removed, what remains may be the fermentation medium with the cells used for producing the intermediate linear alkanoic acid comprising 4 to 7 carbon atoms, e.g. hexanoic acid, and these cells together with the fermentation medium may then be recycled for step (a). A skilled person would be able to determine if there needs to be a replenishment of the fermentation medium and/or cells after the first cycle. In particular, a first cycle according to any aspect of the present invention involves one round of steps (a) to (c). The medium and/or cells may then be recycled from the second cycle onwards. The extractant may comprise a combination of compounds that may result in an efficient means of extracting the hexanoic acid from the aqueous medium. In particular, the extractant may comprise

- at least one alkyl-phosphine oxide and at least one alkane comprising at least 12 carbon atoms; or
- at least one trialkylamine and at least one alkane comprising at least 12 carbon atoms.

The extractant according to any aspect of the present invention may efficiently extract the intermediate linear alkanoic acid comprising 4 to 7 carbon atoms, e.g. hexanoic acid, into the extractant. This extractant of a mixture of alkyl-phosphine oxide or trialkylamine and at least one alkane may be considered suitable in the method according to any aspect of the present invention as the mixture works efficiently in extracting the desired intermediate linear alkanoic acid comprising 4 to 7 carbon atoms, e.g. hexanoic acid, in the presence of a fermentation medium. In particular, the mixture of alkyl-phosphine oxide or trialkylamine and at least one alkane may be considered to work better than any method currently known in the art for extraction of the intermediate linear alkanoic acid comprising 4 to 7 carbon atoms, e.g. hexanoic acid, as it does not require any special equipment to be carried out and it is relatively easy to perform with a high product yield. Further, the extractant according to any aspect of the present invention is also not toxic the microorganism according to step (a).

The alkane in the extractant may comprise at least 12 carbon atoms. In particular, the alkane may comprise at 12-18 carbon atoms. In one example, the alkane may be selected from the group consisting of dodecane, tridecane, tetradecane, pentadecane, hexadecane, heptadecane and octadecane. In a further example, the extractant may comprise a mixture of alkanes.

Alkyl-phosphine oxides have a general formula of $OPX_3$, where X is an alkyl. Suitable alkyl phosphine oxides according to any aspect of the present invention include an alkyl group composed of a linear, branched or cyclic hydrocarbon, the hydrocarbon composed of from 1 to about 100 carbon atoms and from 1 to about 200 hydrogen atoms. In particular, "alkyl" as used in reference to alkyl phosphine oxide according to any aspect of the present invention can refer to a hydrocarbon group having 1 to 20 carbon atoms, frequently between 4 and 15 carbon atoms, or between 6 and 12 carbon atoms, and which can be composed of straight chains, cyclic, branched chains, or mixtures of these. The alkyl phosphine oxide may have from one to three alkyl groups on each phosphorus atom. In one example, the alkyl phosphine oxide has three alkyl groups on P. In some examples, the alkyl group may comprise an oxygen atom in place of one carbon of a C4-C15 or a C6-C12 alkyl group, provided the oxygen atom is not attached to P of the alkyl phosphine oxide.

Typically, the alkyl phosphine oxide is selected from the group consisting of trioctylphosphine oxide, tri-butylphosphine oxide, hexyl-phosphine oxide, octylphosphine oxide and mixtures thereof. Even more in particular, the alkyl phosphine oxide may be trioctylphosphine oxide (TOPO).

Trialkylamines are organic-chemical compounds derived from ammonia ($NH_3$), whose three hydrogen atoms are replaced by alkyl radicals. Examples of trialkylamines are dimethylethylamine, methyldiethylamine, triethylamine,

dimethyl-n-propylamine, dimethyl-i-propylamine, methyldi-n-propylamine, dimethylbutylamine, trioctylamine and the like. In particular, the trialkylamine used in the extractant according to any aspect of the present invention may not be soluble in water and may be trioctylamine.

In one example, the extractant according to any aspect of the present invention may be a combination of alkyl-phosphine oxide or trialkylamine and at least one alkane. In particular, the alkane may comprise at least 12 carbon atoms. More in particular, the alkane may comprise at 12-18 carbon atoms. In one example, the alkane may be selected from the group consisting of dodecane, tridecane, tetradecane, pentadecane, hexadecane, heptadecane and octadecane.

In a further example, the extractant may comprise a mixture of alkanes. Even more in particular, the extractant according to any aspect of the present invention may be a combination of TOPO and tetradecane or hexadecane.

Trioctylphosphine oxide (TOPO) is an organophosphorus compound with the formula $OP(C_8H_{17})_3$. TOPO may be part of the extractant together with at least one alkane according to any aspect of the present invention. In particular, the mixture of TOPO and alkane comprising at least 12 carbon atoms may comprise about 1:100 to 1:10 weight ratio of TOPO relative to the alkane. More in particular, the weight ratio of TOPO to alkane in the extractant according to any aspect of the present invention may be about 1:100, 1:90, 1:80, 1:70, 1:60, 1:50, 1:40, 1:30, 1:25, 1:20, 1:15, or 1:10. Even more in particular, the weight ratio of TOPO to alkane may be selected within the range of 1:90 to 1:10, 1:80 to 1:10, 1:70 to 1:10, 1:60 to 1:10, 1:50 to 1:10, 1:40 to 1:10, 1:30 to 1:10 or 1:20 to 1:10. The weight ratio of TOPO to alkane may be between 1:40 to 1:15 or 1:25 to 1:15. In one example, the weight ratio of TOPO to alkane may be about 1:15. In the example, the alkane may be hexadecane and therefore the weight ratio of TOPO to hexadecane may be about 1:15.

In another example, when the extractant comprises an alkyl-phosphine oxide or a trialkylamine that is more soluble in the alkane used in the extractant compared to the solubility of TOPO in alkane comprising at least 12 carbon atoms, the weight ratio of the alkyl-phosphine oxide (other than TOPO) or a trialkylamine to alkane may be 1:1, 2:1, 3:1, 4:1, 5:1, 6:1, 7:1, 8:1, 9:1 or 10:1. In one example, the extractant may be trihexy-phosphine oxide and the ratio of trihexy-phosphine oxide to alkane may be 1:1. In other examples, the extractant may be a lower chain alkyl-phosphine oxide and the ratio of the lower chain alkyl-phosphine oxide to alkane may be 2:1, 3:1, 4:1, 5:1, 6:1, 7:1, 8:1, 9:1 or 10:1. In this case, a lower-chain alkyl-phosphine oxide refers to a phosphine oxide with a C1-C4 alkyl group. In another example, the extractant may be a trialkylamine, this is known to be more soluble than phosphine oxide in alkanes. For example, the trialkylamine may be a trioctylamine (TOA) that may be present in the extractant according to any aspect of the present invention in the ratio of up to 1:1 with the alkane. Lower chain length amines can be used in even higher ratios. In other examples, the extractant may be a lower chain trialkylamine and the ratio of the lower chain trialkylamine to alkane may be 2:1, 3:1, 4:1, 5:1, 6:1, 7:1, 8:1, 9:1 or 10:1. In this case, a lower-chain alkyl-phosphine oxide refers to a phosphine oxide with a C1-C4 alkyl group.

The term 'about' as used herein refers to a variation within 20 percent. In particular, the term "about" as used herein refers to +/- 20%, more in particular, +/-10%, even more in particular, +/- 5% of a given measurement or value.

Preferably, in step (b) of the method according to the present invention, the the alkyl-phosphine oxide is selected from the group consisting of trioctylphosphine oxide, hexylphosphine oxide, octylphosphine oxide and mixtures thereof, preferably trioctylphosphine oxide (TOPO) and the alkane is selected from the group consisting of pentadecane, hexadecane, heptadecane, octadecane, and tetradecane, preferably tetradecane, preferably in a weight ratio of TOPO to tetradecane between 1:100 to 1:10.

Preferably, the pH of the aqueous medium in step (b) of the method according to the present invention is maintained between 5.5 and 8.

In step (a) according to any aspect of the present invention, ethanol and/or a linear alkanoic acid comprising 2 to 5 carbon atoms, e.g. acetic acid, propanoic acid, butanoic acid or pentanoic acid, or any salts thereof is contacted with at least one microorganism capable of carrying out two-carbon chain elongation to produce as an intermediate a linear alkanoic acid comprising 4 to 7 carbon atoms, e.g. hexanoic acid, butanoic acid or pentanoic acid, and/or a salt thereof and/or an ester thereof.

In one example, the carbon source may be ethanol in combination with at least one other carbon source selected from the group consisting of acetate, propanoate, butanoate (butyrate) and pentanoate. In particular, the carbon source may be ethanol and acetate. In another example, the carbon source may be a combination of ethanol and butyric (butanoic) acid. In one example, the carbon substrate may be ethanol alone. In another example, the carbon substrate may be the linear alkanoic acid comprising 2 to 5 carbon atoms, e.g. acetate, alone.

The source of the linear alkanoic acid comprising 2 to 5 carbon atoms, e.g. acetate, and/or ethanol may vary depending on availability. In one example, the ethanol and/or the linear alkanoic acid comprising 2 to 5 carbon atoms (e.g. acetate) may be the product of fermentation of synthesis gas (syngas) or any carbohydrate known in the art. In particular, the carbon source for the linear alkanoic acid comprising 2 to 5 carbon atoms (e.g. acetate) and/or ethanol production may be selected from the group consisting of alcohols, aldehydes, glucose, sucrose, fructose, dextrose, lactose, xylose, pentose, polyol, hexose, ethanol and synthesis gas. Mixtures of sources can be used as a carbon source.

Even more in particular, the carbon source may be synthesis gas (syngas). The synthesis gas may be converted to ethanol and/or the linear alkanoic acid comprising 2 to 5 carbon atoms, e.g. acetate, in the presence of at least one acetogenic bacteria.

With respect to the source of substrates comprising carbon dioxide and/or carbon monoxide, a skilled person would understand that many possible sources for the provision of CO and/or $CO_2$ as a carbon source exist. The syngas or sources of syngas may be derived for instance from steam reforming, partial oxidation or electrochemical synthesis from water or $CO_2$. It can be seen that in practice, as the carbon source of the present invention any gas or any gas mixture can be used which is able to supply the microorganisms with sufficient amounts of carbon, so that the lower alkanoic acid (e.g. acetate) and/or ethanol, may be formed from the source of CO and/or $CO_2$.

Generally, for the acetogenic cell of the present invention the carbon source comprises at least 50% by weight, at least 70% by weight, particularly at least 90% by weight of $CO_2$ and/or CO, wherein the percentages by weight - % relate to all carbon sources that are available to the cell according to any aspect of the present invention. The carbon material source may be provided.

Examples of carbon sources in gas forms include exhaust gases such as synthesis gas, flue gas and petroleum refinery gases produced by yeast fermentation or clostridial fermentation. These exhaust gases are formed from the gasification of cellulose-containing materials or coal gasification. In one example, these exhaust gases may not necessarily be produced as by-products of other processes but can specifically be produced for use with the mixed culture of the present invention.

According to any aspect of the present invention, the carbon source for the production of the linear alkanoic acid comprising 2 to 5 carbon atoms (e.g. acetate) and/or ethanol used in step (a) according to any aspect of the present invention may be synthesis gas. Synthesis gas can for example be produced as a by-product of coal gasification. Accordingly, the microorganism according to any aspect of the present invention may be capable of converting a substance which is a waste product into a valuable resource.

In another example, synthesis gas may be a by-product of gasification of widely available, low-cost agricultural raw materials for use with the mixed culture of the present invention to produce substituted and unsubstituted organic compounds.

There are numerous examples of raw materials that can be converted into synthesis gas, as almost all forms of vegetation can be used for this purpose. In particular, raw materials are selected from the group consisting of perennial grasses such as miscanthus, corn residues, processing waste such as sawdust and the like.

In general, synthesis gas may be obtained in a gasification apparatus of dried biomass, mainly through pyrolysis, partial oxidation and steam reforming, wherein the primary products of the synthesis gas are CO, $H_2$ and $CO_2$. Syngas may also be a product of electrolysis of $CO_2$. A skilled person would understand the suitable conditions to carry out electrolysis of $CO_2$ to produce syngas comprising CO in a desired amount.

Usually, a portion of the synthesis gas obtained from the gasification process is first processed in order to optimize product yields, and to avoid formation of tar. Cracking of the undesired tar and CO in the synthesis gas may be carried out using lime and/or dolomite.

The overall efficiency, ethanol and/or acetate productivity and/or overall carbon capture of the method of the present invention may be dependent on the stoichiometry of the $CO_2$, CO, and $H_2$ in the continuous gas flow. The continuous gas flows applied may be of composition $CO_2$ and $H_2$. In particular, in the continuous gas flow, concentration range of $CO_2$ may be about 10-50 %, in particular 3 % by weight and $H_2$ would be within 44 % to 84 %, in particular, 64 to 66.04 % by weight. In another example, the continuous gas flow can also comprise inert gases like $N_2$, up to a $N_2$ concentration of 50 % by weight.

More in particular, the carbon source comprising CO and/or $CO_2$ contacts the acetogenic cells in a continuous gas flow. Even more in particular, the continuous gas flow comprises synthesis gas. These gases may be supplied for example using nozzles that open up into the aqueous medium, frits, membranes within the pipe supplying the gas into the aqueous medium and the like.

A skilled person would understand that it may be necessary to monitor the composition and flow rates of the streams at relevant intervals. Control of the composition of the stream can be achieved by varying the proportions of the constituent streams to achieve a target or desirable composition. The composition and flow rate of the blended stream can be monitored by any means known in the art. In one example, the system is adapted to continuously monitor the flow rates and compositions of at least two streams and combine them to produce a single blended substrate stream in a continuous gas flow of optimal composition and means for passing the optimized substrate stream to the fermenter.

According to any aspect of the present invention, a reducing agent, for example hydrogen may be supplied together with the carbon source. In particular, this hydrogen may be supplied when the CO and/or $CO_2$ is supplied and/or used. In one example, the hydrogen gas is part of the synthesis gas present according to any aspect of the present invention. In another example, where the hydrogen gas in the synthesis gas is insufficient for the method of the present invention, additional hydrogen gas may be supplied.

The term "acetogenic bacteria" as used herein refers to a microorganism which is able to perform the Wood-Ljungdahl pathway and thus is able to convert CO, $CO_2$ and/or hydrogen to a lower alkanoic acid, e.g. acetate. These microorganisms include microorganisms which in their wild-type form do not have a Wood-Ljungdahl pathway, but have acquired this trait as a result of genetic modification. Such microorganisms include but are not limited to E. *coli* cells. These microorganisms may be also known as carboxydotrophic bacteria. Currently, 21 different genera of the acetogenic bacteria are known in the art (Drake et al., 2006), and these may also include some clostridia (Drake & Kusel, 2005). These bacteria are able to use carbon dioxide or carbon monoxide as a carbon source with hydrogen as an energy source (Wood, 1991). Further, alcohols, aldehydes, carboxylic acids as well as numerous hexoses may also be used as a carbon source (Drake et al., 2004). The reductive pathway that leads to the formation of acetate is referred to as acetyl-CoA or Wood-Ljungdahl pathway.

In particular, the acetogenic bacteria may be selected from the group consisting of *Acetoanaerobium notera (ATCC 35199*), *Acetonema longum (DSM 6540), Acetobacterium carbinolicum (DSM 2925), Acetobacterium malicum (DSM 4132), Acetobacterium species no. 446 (*Morinaga et al., 1990, J. Biotechnol., Vol. 14, p. 187-194*),Acetobacterium wieringae (DSM 1911), Acetobacterium woodii (DSM 1030), Alkalibaculum bacchi (DSM 22112), Archaeoglobus fulgidus (DSM 4304), Blautia producta (DSM 2950, formerly Ruminococcus productus, formerly Peptostreptococcus productus), Butyribacterium methylotrophicum (DSM 3468), Clostridium aceticum (DSM 1496), Clostridium autoethanogenum (DSM 10061, DSM 19630 and DSM 23693), Clostridium carboxidivorans (DSM 15243), Clostridium coskatii (ATCC no. PTA-10522), Clostridium drakei (ATCC BA-623), Clostridium formicoaceticum (DSM 92), Clostridium glycolicum (DSM 1288), Clostridium ljungdahlii (DSM 13528), Clostridium ljungdahlii C-01 (ATCC 55988), Clostridium ljungdahlii ERI-2 (ATCC 55380), Clostridium ljungdahlii O-52 (ATCC 55989), Clostridium mayombei (DSM 6539), Clostridium methoxybenzovorans (DSM 12182), Clostridium ragsdalei (DSM 15248), Clostridium scatologenes (DSM 757), Clostridium species ATCC 29797 (*Schmidt et al., 1986, Chem. Eng. Commun., Vol. 45, p. 61-73*), Desulfotomaculum kuznetsovii (DSM 6115), Desulfotomaculum thermobezoicum subsp. thermosyntrophicum (DSM 14055), Eubacterium limosum (DSM 20543), Methanosarcina acetivorans C2A (DSM 2834), Moorella sp. HUC22-1 (*Sakai et al., 2004, Biotechnol. Let., Vol. 29, p. 1607-1612*), Moorella thermoacetica (DSM 521, formerly Clostridium thermoaceticum), Moorella thermoautotrophica (DSM 1974), Oxobacter pfennigii (DSM 322), Sporomusa aerivorans (DSM 13326), Sporomusa ovata (DSM 2662), Sporomusa silvacetica (DSM 10669), Sporomusa sphaeroides (DSM 2875), Sporomusa termitida (DSM 4440) and Thermoanaerobacter kivui (DSM 2030, formerly Acetogenium kivui).*

More in particular, the strain ATCC BAA-624 of *Clostridium carboxidivorans* may be used. Even more in particular, the bacterial strain labelled "P7" and "P11" of *Clostridium carboxidivorans* as described for example in U.S. 2007/0275447 and U.S. 2008/0057554 may be used.

Another particularly suitable bacterium may be *Clostridium ljungdahlii.* In particular, strains selected from the group consisting of *Clostridium ljungdahlii* PETC, *Clostridium ljungdahlii* ERI2, *Clostridium ljungdahlii* COL and *Clostridium ljungdahlii* O-52 may be used in the conversion of synthesis gas to hexanoic acid. These strains for example are described in WO 98/00558, WO 00/68407, ATCC 49587, ATCC 55988 and ATCC 55989.

In one example, the production of the intermediate linear alkanoic acid comprising 4 to 7 carbon atoms, its salt and/or ester thereof, e.g. hexanoic acid, is from the linear alkanoic acid comprising 2 to 5 carbon atoms (e.g. acetate) and/or ethanol which is from synthesis gas and may involve the use of the acetogenic bacteria in conjunction with a microorganism capable of carbon chain elongation. For example, *Clostridium ljungdahlii* may be used simultaneously with *Clostridium kluyveri.* In another example, a single acetogenic cell may be capable of the activity of both organisms. For example, the acetogenic bacteria may be C. *carboxidivorans* which may be capable of carrying out both the Wood-Ljungdahl pathway and the carbon chain elongation pathway.

For example, ethanol and/or acetate used in step (a) according to any aspect of the present invention may be a product of fermentation of synthesis gas or may be obtained through other means. The ethanol and/or acetate may then be brought into contact with the microorganism in step (a).

The term "contacting", as used herein, means bringing about direct contact between the microorganism and the ethanol and/or the linear alkanoic acid comprising 2 to 5 carbon atoms, e.g. acetate. In one example, ethanol is the carbon source and the contacting in step (a) involves contacting the ethanol with the microorganism of step (a). The contact may be a direct contact or an indirect one that may include a membrane or the like separating the cells from the ethanol or where the cells and the ethanol may be kept in two different compartments etc. For example, in step (b) the intermediate linear alkanoic acid comprising 4 to 7 carbon atoms, its salt and/or ester thereof, e.g. the hexanoic acid, and the extracting medium may be in different compartments.

The microorganisms capable of producing the intermediate linear alkanoic acid comprising 4 to 7 carbon atoms, its salt and/or ester thereof, e.g. the hexanoic acid, according to any aspect of the present invention may be cultivated with any culture media, substrates, conditions, and processes generally known in the art for culturing bacteria. This allows for the the intermediate linear alkanoic acid comprising 4 to 7 carbon atoms, its salt and/or ester thereof (e.g. hexanoic acid) to be produced using a biotechnological method. Depending on the microorganism that is used for the production of the intermediate linear alkanoic acid comprising 4 to 7 carbon atoms, its salt and/or ester thereof (e.g. hexanoic acid), appropriate growth medium, pH, temperature, agitation rate, inoculum level, and/or aerobic, microaerobic,

or anaerobic conditions are varied. A skilled person would understand the other conditions necessary to carry out the method according to any aspect of the present invention. In particular, the conditions in the container (e.g. fermenter) may be varied depending on the microorganisms used. The varying of the conditions to be suitable for the optimal functioning of the microorganisms is within the knowledge of a skilled person.

In one example, the method, in particular step (a) according to any aspect of the present invention may be carried out in an aqueous medium with a pH between 5 and 8, 5.5 and 8 or 5.5 and 7. The pressure may be between 1 and 10 bar. The microorganisms may be cultured at a temperature ranging from about 20° C to about 80° C. In one example, the microorganism may be cultured at 37° C.

In some examples, for the growth of the microorganism and for its production of the intermediate linear alkanoic acid comprising 4 to 7 carbon atoms, its salt and/or ester thereof (e.g. hexanoic acid), the aqueous medium may comprise any nutrients, ingredients, and/or supplements suitable for growing the microorganism or for promoting the production of the intermediate linear alkanoic acid comprising 4 to 7 carbon atoms, its salt and/or ester thereof, e.g. the hexanoic acid. In particular, the aqueous medium may comprise at least one of the following: carbon sources, nitrogen sources, such as an ammonium salt, yeast extract, or peptone; minerals; salts; cofactors; buffering agents; vitamins; and any other components and/or extracts that may promote the growth of the bacteria. The culture medium to be used must be suitable for the requirements of the particular strains. Descriptions of culture media for various microorganisms are given in "Manual of Methods for General Bacteriology".

The term "an aqueous solution" or "medium" comprises any solution comprising water, mainly water as solvent that may be used to keep the cell according to any aspect of the present invention, at least temporarily, in a metabolically active and/or viable state and comprises, if such is necessary, any additional substrates. The person skilled in the art is familiar with the preparation of numerous aqueous solutions, usually referred to as media that may be used to keep and/or culture the cells, for example LB medium in the case of E. *coli,* ATCC1754-Medium may be used in the case of C. *ljungdahlii.* It is advantageous to use as an aqueous solution a minimal medium, i.e. a medium of reasonably simple composition that comprises only the minimal set of salts and nutrients indispensable for keeping the cell in a metabolically active and/or viable state, by contrast to complex mediums, to avoid dispensable contamination of the products with unwanted side products. For example, M9 medium may be used as a minimal medium. The cells are incubated with the carbon source sufficiently long enough to produce the desired product. For example, for at least 1, 2, 4, 5, 10 or 20 hours. The temperature chosen must be such that the cells according to any aspect of the present invention remains catalytically competent and/or metabolically active, for example 10 to 42 °C, preferably 30 to 40 °C, in particular, 32 to 38 °C in case the cell is a C. *ljungdahlii* cell. The aqueous medium according to any aspect of the present invention also includes the medium in which the hexanoic acid is produced. It mainly refers to a medium where the solution comprises substantially water. In one example, the aqueous medium in which the cells are used to produce the hexanoic acid is the very medium which contacts the extractant for extraction of the hexanoic acid.

According to any aspect of the present invention it is preferred that the extraction is carried out in step (b) while fermentation takes place in step (a) simultaneously. This depicts an *in situ* extracting the intermediate linear alkanoic acid comprising 4 to 7 carbon atoms, its salt and/or ester thereof, e.g. the hexanoic acid and/or ester thereof, in step (b).

In step (b) according to any aspect of the present invention, the intermediate linear alkanoic acid comprising 4 to 7 carbon atoms, its salt and/or ester thereof (e.g. the hexanoic acid) in the aqueous medium may contact the extractant for a time sufficient to extract the hexanoic acid from the aqueous medium into the extractant. A skilled person may be capable of determining the amount of time needed to reach distribution equilibrium and the right bubble agglomeration that may be needed to optimize the extraction process. In some examples the time needed may be dependent on the amount of hexanoic acid that may be extracted. In particular, the time needed to extract the intermediate linear alkanoic acid comprising 4 to 7 carbon atoms, its salt and/or ester thereof (e.g. the hexanoic acid) from the aqueous medium into the extractant may only take a few minutes. According to any aspect of the present invention, where the extraction is carried out in step (b) as fermentation takes place in step (a), the time for extraction may be equivalent to the time of fermentation.

The ratio of the extractant used to the amount of hexanoic acid to be extracted may vary depending on how quick the extraction is to be carried out. In one example, the amount of extractant is equal to the amount of aqueous medium comprising the intermediate linear alkanoic acid comprising 4 to 7 carbon atoms, its salt and/or ester thereof, e.g. the hexanoic acid. After the step of contacting the extractant with the aqueous medium, the two phases (aqueous and organic) are separated using any means known in the art. In one example, the two phases may be separated using a separation funnel. The two phases may also be separated using mixer-settlers, pulsed columns, and the like. In one example, the separation of the extracting medium from the intermediate linear alkanoic acid comprising 4 to 7 carbon atoms, its salt and/or ester thereof, e.g. the hexanoic acid, may be carried out using distillation in view of the fact that the intermediate linear alkanoic acid comprising 4 to 7 carbon atoms, e.g. hexanoic acid, distills at a significantly lower boiling point than the extractant. A skilled person may be able to select the best method of separating the extractant from the desired hexanoic acid in step (b) depending on the characteristics of the the intermediate linear alkanoic acid comprising 4 to 7 carbon atoms, its salt and/or ester thereof, e.g. hexanoic acid. In particular, step (c) according to any

aspect of the present invention involves the recovering of the intermediate linear alkanoic acid comprising 4 to 7 carbon atoms, its salt and/or ester thereof, e.g. the hexanoic acid, from step (b). The intermediate linear alkanoic acid comprising 4 to 7 carbon atoms, its salt and/or ester thereof, e.g. hexanoic acid, brought into contact with the organic extractant results in the formation of two phases, the two phases (aqueous and organic) are separated using any means known in the art. In one example, the two phases may be separated using a separation funnel. The two phases may also be separated using mixer-settlers, pulsed columns, thermal separation and the like. In one example, where the intermediate linear alkanoic acid comprising 4 to 7 carbon atoms is hexanoic acid, the separation of the extracting medium from the hexanoic acid may be carried out using distillation in view of the fact that hexanoic acid distills at a significantly lower boiling point than the extracting medium.

Step (b) ends with the organic absorbent made available again to be recycled or reused.

Accordingly, the method of extraction of hexanoic acid according to any aspect of the present invention may be used together with any biotechnological method of producing the hexanoic acid. This is especially advantageous as usually during the fermentation process to produce hexanoic acid using biological methods, the hexanoic acid would be left to collect in the aqueous medium and after reaching certain concentrations in the fermentation medium, the very target product (hexanoic acid) may inhibit the activity and productivity of the microorganism. This thus limits the overall yield of the fermentation process. With the use of this extraction method, the intermediate linear alkanoic acid comprising 4 to 7 carbon atoms, its salt and/or ester thereof, e.g. the hexanoic acid, is extracted as it is produced thus reducing end-product inhibition drastically.

The method according to any aspect of the present invention is also more efficient and cost-effective than the traditional methods of removing hexanoic acid, particularly from a fermentation method as it is produced, as there is no primary reliance on distillation and/or a precipitation for recovering of hexanoic acids. Distillation or precipitation process may lead to higher manufacturing costs, lower yield, and higher waste products therefore reducing the overall efficiency of the process. The method according to any aspect of the present invention attempts to overcome these shortcomings.

In particular, the mixture of the microorganism and the carbon source according to any aspect of the present invention may be employed in any known bioreactor or fermenter to carry out any aspect of the present invention. In one example, the complete method according to any aspect of the present invention that begins with the biotechnological production of the intermediate linear alkanoic acid comprising 4 to 7 carbon atoms, its salt and/or ester thereof, e.g. the hexanoic acid from acetate and/or ethanol and ends with the extraction of the the intermediate linear alkanoic acid comprising 4 to 7 carbon atoms, its salt and/or ester thereof, e.g. hexanoic acid, takes place in a single container. There may therefore be no separation step between the step of producing the intermediate linear alkanoic acid comprising 4 to 7 carbon atoms, its salt and/or ester thereof, e.g. hexanoic acid, and the step of extracting the intermediate linear alkanoic acid comprising 4 to 7 carbon atoms, its salt and/or ester thereof, e.g. the hexanoic acid. This saves time and costs. In particular, during the fermentation process, the microorganism may be grown in the aqueous medium and in the presence of the extractant. The method according to any aspect of the present invention thus provides for a one pot means of producing the intermediate linear alkanoic acid comprising 4 to 7 carbon atoms, its salt and/or ester thereof, e.g. of the hexanoic acid. Also, since the intermediate linear alkanoic acid comprising 4 to 7 carbon atoms, its salt and/or ester thereof, e.g. the hexanoic acid, is being extracted as it is produced, no end-product inhibition takes place, ensuring that the yield of hexanoic acid is maintained. A further step of separation may be carried out to remove the intermediate linear alkanoic acid comprising 4 to 7 carbon atoms, its salt and/or ester thereof, e.g. the hexanoic acid. Any separation method known in the art such as using a funnel, column, distillation and the like may be used. The remaining extractant and/or the cells may then be recycled.

In another example, the extraction process of the intermediate linear alkanoic acid comprising 4 to 7 carbon atoms, its salt and/or ester thereof, e.g. the hexanoic acid, may take place as a separate step and/or in another pot. After fermentation has taken place, where the desired intermediate linear alkanoic acid comprising 4 to 7 carbon atoms, e.g. hexanoic acid, to be extracted has already been produced, the extractant according to any aspect of the present invention may be added to the fermentation medium or the fermentation medium may be added to a pot comprising the extractant. The desired intermediate linear alkanoic acid comprising 4 to 7 carbon atoms, e.g. hexanoic acid, may then be extracted by any separation method known in the art such as using a funnel, column, distillation and the like. The remaining extractant may then be recycled. The fermentation medium with the cells may also be recycled.

Another advantage of the method is that the extractant may be recycled. Therefore, once the intermediate linear alkanoic acid comprising 4 to 7 carbon atoms, e.g. hexanoic acid, is separated from extractant, the extractant can be recycled and reused, reducing waste.

Step (c) of the method according to any aspect of the present invention involves (c) contacting the extracted intermediate linear alkanoic acid comprising 4 to 7 carbon atoms, its salt and/or ester thereof, e.g. the hexanoic acid, from (b) with at least one ketonization catalyst and optionally a further alkanoic acid comprising 2 to 22 carbon atoms under suitable reaction conditions for chemical ketonization of the intermediate linear alkanoic acid comprising 4 to 7 carbon atoms, its salt and/or ester thereof, e.g. the hexanoic acid, to a linear alkanone comprising 7 to 28 carbon atoms, preferably 6-undecanone.

The term "further alkanoic acid comprising 2 to 22 carbon atoms" does not encompass hexanoic acid. Preferably, the further alkanoic acid comprising 2 to 22 carbon atoms is selected from straight chain alkanoic acids comprising 4 to 18, preferably 5 to 12, carbon atoms.

A ketonization catalyst according to any aspect of the present invention may be any metal oxide catalyst or mixtures thereof. Ketonization reacts the intermediate linear alkanoic acid comprising 4 to 7 carbon atoms, its salt and/or ester thereof, e.g. the hexanoic acid, with the further alkanoic acid and/or the intermediate linear alkanoic acid comprising 4 to 7 carbon atoms, its salt and/or ester thereof, e.g. the hexanoic acid, preferably dimerizes two molecules of the intermediate linear alkanoic acid comprising 4 to 7 carbon atoms (e.g. hexanoic acid), to one ketone molecule with the removal of one water and one carbon dioxide. In case of hexanoic acid being the intermediate linear alkanoic acid comprising 4 to 7 carbon atoms, the mechanism that may be involved in ketonization of the intermediate linear alkanoic acid comprising 4 to 7 carbon atoms where hexanoic anhydride ($(CH_3(CH_2)_4)COOCO(CH_2)_4CH_3$) may be formed is disclosed at least in Woo, Y., Ind. Eng. Chem. Res. 2017, 56: 872-880. Ketonization of hexanoic acid in the presence of a variety of metal oxide catalysts is at also shown in Wang, S. J. Phys. Chem. C 2017, 121, 18030-18046.

The ketonization catalyst used according to any aspect of this present invention may be a heterogeneous catalyst for the efficient production of a higher-energy-density ketone, preferably C11, from biologically produced hexanoic acid according to step (a). In particular, a ketonization catalyst may be any metal oxide catalyst or mixtures thereof selected from the group consisting of metal oxide catalyst or mixtures thereof is selected from the group consisting of heteropoly acid ($H_3PW_{12}O_{40}$) catalyst, niobium oxide ($Nb_2O_5$) catalyst, titanium oxide ($TiO_2$) catalyst, cerium oxide ($CeO_2$) catalyst, zinc-chromium (Zn-Cr) mixed oxide catalyst, manganese oxide ($MnO_x$) catalyst, lanthanum oxide ($La_2O_3$) catalyst, magnesium oxide (MgO) catalyst, iron oxide (FeO, $FeO_2$, FezOs, $Fe_3O_4$, $Fe_4O_5$, $Fe_5O_6$, $Fe_5O_7$), silicon-aluminium ($Si_yAl_zO$) mixed oxide catalyst, aluminium oxide ($Al_2O_3$) catalyst and zirconia ($ZrO_2$) catalyst. The 'x' in $MnO_x$ may be 1, 2 or 4. The 'y' and 'z' in $Si_yAl_zO$ may refer to any number where the ratio z/y is any number between 0 to 1. In one example, ketonization is carried out on hexanoic acid as disclosed in Pham T. N., ACS Catal. 2013, 3: 2456-2473 using a suitable heterogenous hydrogenation metal catalyst and suitable reaction conditions. The conditions as disclosed can vary depending on the catalyst used for effective yield of linear alkanones comprising 7 to 28 carbon atoms, e.g. 6-undecanone. In yet another example, $MnO_2$ and/or $Al_2O_3$ catalyst may be used based on what is disclosed in Gliriski, M. et al, Polish J. Chem. 2004, 78: 299-302 for ketonizing hexanoic acid to 6-undecanone. In a further example, $Nb_2O_5$ catalyst may be used as disclosed in US 6,265,618 B1 especially in example 3 in ketonizing hexanoic acid to 6-undecanone. A skilled person would by simple trial and error be able to identify the suitable catalyst and the appropriate conditions for producing linear alkanones comprising 7 to 28 carbon atoms, preferably 6-undecanone, from hexanoic acid and eventually another alkanoic acid based on the state of the art. Orozco, L.M et al ChemSusChem, 2016, 9(17): 2430-2442 and Orozco, L.M et al Green Chemistry, 2017, 19(6): 1555-1569 also disclose other catalyst that may be used as ketonization catalysts according to any aspect of the present invention.

The metal oxide catalyst or mixtures thereof is preferably selected from the group consisting of heteropoly acid ($H_3PW_{12}O_{40}$) catalyst, titanium oxide ($TiO_2$) catalyst, cerium oxide ($CeO_2$) catalyst, zinc-chromium (Zn-Cr) mixed oxide catalyst, manganese oxide ($MnO_2$) catalyst, lanthanum oxide ($La_2O_3$) catalyst, magnesium oxide (MgO) catalyst, iron oxide (FeO, $FeO_2$, FezOs, $Fe_3O_4$, $Fe_4O_5$, $Fe_5O_6$, $Fe_5O_7$), silicon-aluminium (Si-Al) mixed oxide catalyst and zirconia ($ZrO_2$) catalyst. Preferably, the ketonization catalyst in step (c) is a zirconia aerogel catalyst.

It would be within the knowledge of a skilled person to determine the suitable conditions for the use of the different ketonization catalysts in step (c). In particular, the ketonization catalyst may be a zirconia aerogel catalyst and may be used in the ketonization of hexanoic acid as disclosed in Woo, Y., Ind. Eng. Chem. Res. 2017, 56: 872-880. The zirconia aerogel catalyst may not only efficiently produce linear alkanones comprising 7 to 28 carbon atoms, preferably 6-undecanone, but it also avoids leaching of the catalysts. Lee, Y. et al in Applied Catalysis A: General. 2015, 506: 288-293 discloses different ketonization catalysts and their effectiveness in ketonization hexanoic acid. A skilled person can very easily use the method described in Lee Y., et al to determine the suitable ketonization catalysts and/or conditions for use in the ketonization of the intermediate linear alkanoic acid comprising 4 to 7 carbon atoms, e.g. hexanoic acid.

In particular, suitable reaction conditions of step (c) comprises reaction temperatures of 100°C - 500°C, 100°C - 450°C, 100°C - 400°C, 100°C - 350°C, 100°C - 300°C, 100°C - 250°C, 100°C - 200°C, 150°C - 500°C, 150°C - 450°C, 150°C - 400°C, 150°C - 350°C, 150°C - 300°C, 150°C - 250°C, 150°C - 200°C, 200°C - 500°C, 200°C - 450°C, 200°C - 400°C, 200°C - 350°C, 200°C - 300°C, 200°C - 250°C, 250°C - 500°C, 250°C - 450°C, 250°C - 400°C, 250°C - 350°C, 250°C - 300°C and the like.

Preferably, the suitable reaction conditions of step (c) comprises reaction temperatures of 150°C - 350 °C.

More in particular, linear alkanones comprising 7 to 28 carbon atoms, preferably 6-undecanone, may be produced from hexanoic acid using $MgO/SiO_2$ catalyst as the ketonization catalyst at reaction temperatures of 150°C - 350°C, preferably at reaction temperatures of 200° C - 350° C.

Step (d) of the method according to the present invention provides contacting the linear alkanone comprising 7 to 28 carbon atoms, preferably 6-undecanone, obtained in step (c) with at least one hydrogenation metal catalyst for catalytic hydrogenation of the linear alkanone comprising 7 to 28 carbon atoms, preferably 6-undecanone, to a corresponding linear alkanol comprising 7 to 28 carbon atoms, preferably 6-undecanol.

In particular step (d) of the method according to the present invention comprises contacting the alkanone comprising 7 to 28 carbon atoms, preferably the 6-undecanone, produced according to any aspect of the present invention with at least one hydrogenation metal catalyst for catalytic hydrogenation of the alkanone comprising 7 to 28 carbon atoms, preferably 6-undecanone, to secondary linear alkanols comprising 7 to 28 carbon atoms, preferably 6-undecanol. Linear alkanol comprising 7 to 28 carbon atoms, preferably 6-undecanol ($C_{11}H_{24}O$), a secondary alcohol, are a result of the catalytic hydrogenation of the alkanone comprising 7 to 28 carbon atoms, preferably 6-undecanone, a molecule of hydrogen is added across the carbon-oxygen double bond to ultimately furnish the secondary linear alkanols comprising 7 to 28 carbon atoms , preferably 6-undecanol, as the final product.

The hydrogenation metal catalyst may be a homogeneous or heterogeneous catalyst. Homogeneous metal catalysts may be metal complexes that are known in the art. In particular, the hydrogenation metal catalyst may be a heterogeneous catalyst. Some advantages of using multiphase catalytic reactions using solid catalysts include easy separation of catalysts and products, easy recovery, and catalyst recycling, and relatively mild operating conditions. There are also clear economic and environmental incentives in using heterogeneous catalysts. In particular, the hydrogenation metal catalyst may be selected from the group consisting of ruthenium (Ru) catalyst, rhenium (Re) catalyst, nickel (Ni) catalyst, iron (Fe), cobalt (Co), palladium (Pd) catalyst and platinum (Pt) catalyst. More in particular, the catalyst may be selected from the group consisting of Ni, Pd and Pt catalyst. In one example, the hydrogenation metal catalyst used according to any aspect of the present invention may be nickel nanoparticles as described in Alonso, F. Tetrahedron, 2008, 64: 1847-52. In another example, Iron(II) PNP Pincer Complexes may be used as the hydrogenation metal catalyst for hydrogenation of the linear alkanone comprising 7 to 28 carbon atoms to the linear secondary alcohol comprising 7 to 28 carbon atoms, preferably from 6-undecanone to 6-undecanol, as disclosed in Gorgas, N., Organometallics, 2014, 33 (23): 6905-6914. In yet another example, magnetite nanoparticles of ruthenium (Ru) catalyst, rhenium (Re) catalyst, nickel (Ni) catalyst, iron (Fe), cobalt (Co), palladium (Pd) catalyst or platinum (Pt) catalyst as described in Tariq Shah M., et al., ACS Applied Materials & Interfaces, 2015: 7(12), 6480-9 may be used as the heterogenous hydrogenation metal catalyst according to any aspect of the present invention.

In yet another example, a copper-phosphine complex is used as a homogeneous hydrogenation metal catalyst according to any aspect of the present invention as disclosed in Chen, J-X., Tetrahedron, 2000, 56: 2153-2166. In a further example, a heterogenous Pt catalyst, in particular a $Pt/Al_2O_3$ catalyst, as disclosed in Journal of Molecular Catalysis A: Chemical, 2014, 388-389: 116-122 may be used in hydrogenation of the linear alkanone comprising 7 to 28 carbon atoms to the linear secondary alcohol comprising 7 to 28 carbon atoms, preferably from 6-undecanone to 6-undecanol. ChemSusChem, 2017: 10(11), 2527-2533 also discloses a variety of heterogenous catalysts such as Pt/C, Ru/C, and Pd/C that may be used in combination with or without an acid catalyst for the hydrogenation of 6-undecanone to 6-undecanol. Based on the above, a skilled person may determine a suitable hydrogenation catalyst to be used according to any aspect of the present invention to yield the secondary linear alkanol comprising 7 to 28 carbon atoms from the linear alkanone comprising 7 to 28 carbon atoms, preferably 6-undecanol from 6-undecanone.

A skilled person would easily be able to determine the suitable hydrogenation metal catalyst and vary the conditions accordingly to efficiently produce secondary linear alkanols comprising 7 to 28 carbon atoms, preferably 6-undecanol, from hydrogenation of 6-undecanone.

The hydrogenation metal catalyst of step (d) of the method according to the present invention is preferably selected from the group consisting of ruthenium (Ru) catalyst, rhenium (Re) catalyst, nickel (Ni) catalyst, iron (Fe), cobalt (Co) and platinum (Pt) catalyst.

Step (e) of the method according to the present invention comprises a dehydration of the secondary linear alkanol comprising 7 to 28 carbon atoms, e.g. 6-undecanol in the presence of an acidic heterogeneous catalyst to form a corresponding linear alkene comprising 7 to 28 carbon atoms, e.g. 5-undecene.

Preferably, the acidic heterogeneous catalyst of step (e) is a catalyst comprising an aluminosilicate zeolite. In a particularly preferred embodiment of the method according to the present invention the acidic heterogeneous catalyst of step (e) comprises ZSM-5.

ZSM-5, Zeolite Socony Mobil-5 (framework type MFI from ZSM-5 (five)), is an aluminosilicate zeolite belonging to the pentasil family of zeolites. Its chemical formula is $Na_nAl_nSi_{96-n}O_{192}\cdot 16H_2O$ ($0<n<27$). Patented by Mobil Oil Company in 1975, it is widely used in the petroleum industry as a heterogeneous catalyst for hydrocarbon isomerization reactions.

The acidic heterogeneous catalyst of step (e) may further comprise a transition metal, selected from palladium, nickel, cobalt, iron, molybdenum and manganese, preferably molybdenum, preferably 5 % molybdenum by weight.

The reaction temperature of the dehydration step (e) is from about 100 to about 250 °C, preferably from about 120 to about 180 °C. The solvent may be toluene.

Step (f) of the method according to the present invention comprises a hydroxy or alkoxy carbonylation of the linear alkene comprising 7 to 28 carbon atoms, preferably of 5-undecene, to a linear fatty acid comprising 7 to 28 carbon atoms, preferably lauric acid (dodecanoic acid), or ester in the presence of carbon monoxide, of an acid and of a catalyst

comprising a transition metal, preferably palladium. The transition metal in the catalyst of step (f) has preferably phosphine ligands, preferably (2,2'-[1,2-Phenylenebis[methylene[(1,1-dimethylethyl)phosphinidene]]]bis[pyridine].

Preferably the acid in step (f) is acetic acid or p-toluene sulfonic acid.

The solvent may be water or methanol. The reaction temperature of step (f) is preferably about 120° C.

## EXAMPLES

### Example 1

*Clostridium kluyveri forming hexanoic acid from acetate and ethanol*

For the biotransformation of ethanol and acetate to hexanoic acid the bacterium *Clostridium kluyveri* was used. All cultivation steps were carried out under anaerobic conditions in pressure-resistant glass bottles that can be closed airtight with a butyl rubber stopper.

For the preculture 100 ml of DMSZ52 medium (pH = 7.0; 10 g/L K-acetate, 0.31 g/L $K_2HPO_4$, 0.23 g/L $KH_2PO_4$, 0.25 g/l $NH_4Cl$, 0.20 g/l $MgSO_4 \times 7\ H_2O$, 1 g/L yeast extract, 0.50 mg/L resazurin, 10 μl/l HCl (25%, 7.7 M), 1.5 mg/L $FeCl_2 \times 4H_2O$, 70 μg/L $ZnCl_2 \times 7H_2O$, 100 μg/L $MnCl_2 \times 4H_2O$, 6 μg/L $H_3BO_3$, 190 μg/L $CoCl_2 \times 6H_2O$, 2 μg/L $CuCl_2 \times 6H_2O$, 24 μg/L $NiCl_2 \times 6H_2O$, 36 μg/L $Na_2MO_4 \times 2H_2O$, 0.5 mg/L NaOH, 3 μg/L $Na_2SeO_3 \times 5H_2O$, 4 μg/L $Na_2WO_4 \times 2H_2O$, 100 μg/L vitamin B12, 80 μg/L p-aminobenzoic acid, 20 μg/L D(+) Biotin, 200 μg/L nicotinic acid, 100 μg/L D-Ca-pantothenate, 300 μg/L pyridoxine hydrochloride, 200 μg/l thiamine -$HCl \times 2H_2O$, 20 ml/L ethanol, 2.5 g/L NaHCOs, 0.25 g/L cysteine-$HCl \times H_2O$, 0.25 g/L $Na_2S \times 9H_2O$) in a 250 ml bottle were inoculated with 5 ml of a frozen cryoculture of *Clostridium kluyveri* and incubated at 37°C for 144 h to an $OD_{600nm}$ >0.2. For the main culture 200 ml of fresh DMSZ52 medium in a 500 ml bottle were inoculated with centrifuged cells from the preculture to an $OD_{600nm}$ of 0.1. This growing culture was incubated at 37°C for 27 h to an $OD_{600nm}$ >0.6. Then the cell suspension was centrifuged, washed with production buffer (pH 6.0; 0.832 g/L K-acetate, 5.0 g/l ethanol) and centrifuged again.

For the production culture, 200 ml of production buffer in a 500 ml bottle was inoculated with the washed cells from the main culture to an $OD_{600nm}$ of 0.2. The culture was capped with a butyl rubber stopper and incubated for 71h at 37°C and 100 rpm in an open water shaking bath. At the start and end of the culturing period, samples were taken. These were tested for optical density, pH and the different analytes (tested by NMR).

The results showed that in the production phase the amount of acetate decreased from 0.54 g/l to 0.03 g/l and the amount of ethanol decreased from 5.6 g/l to 4.9 g/l. Also, the concentration of butyric acid was increased from 0.05 g/l to 0.28 g/l and the concentration of hexanoic acid was increased from 0.03 g/l to 0.79 g/l.

### Example 2

*Clostridium kluyveri forming hexanoic acid from butyric acid and ethanol*

For the biotransformation of ethanol and butyric acid to hexanoic acid the bacterium *Clostridium kluyveri* was used. All cultivation steps were carried out under anaerobic conditions in pressure-resistant glass bottles that can be closed airtight with a butyl rubber stopper.

For the preculture 100 ml of DMSZ52 medium (pH = 7.0; 10 g/L K-acetate, 0.31 g/L $K_2HPO_4$, 0.23 g/L $KH_2PO_4$, 0.25 g/l $NH_4Cl$, 0.20 g/l $MgSO_4 \times 7\ H_2O$, 1 g/L yeast extract, 0.50 mg/L resazurin, 10 μl/l HCl (25%, 7.7 M), 1.5 mg/L $FeCl_2 \times 4H_2O$, 70 μg/L $ZnCl_2 \times 7H_2O$, 100 μg/L $MnCl_2 \times 4H_2O$, 6 μg/L $H_3BO_3$, 190 μg/L $CoCl_2 \times 6H_2O$, 2 μg/L $CuCl_2 \times 6H_2O$, 24 μg/L $NiCl_2 \times 6H_2O$, 36 μg/L $Na_2MO_4 \times 2H_2O$, 0.5 mg/L NaOH, 3 μg/L $Na_2SeO_3 \times 5H_2O$, 4 μg/L $Na_2WO_4 \times 2H_2O$, 100 μg/L vitamin B12, 80 μg/L p-aminobenzoic acid, 20 μg/L D(+) Biotin, 200 μg/L nicotinic acid, 100 μg/L D-Ca-pantothenate, 300 μg/L pyridoxine hydrochloride, 200 μg/l thiamine -$HCl \times 2H_2O$, 20 ml/L ethanol, 2.5 g/L NaHCOs, 0.25 g/L cysteine-$HCl \times H_2O$, 0.25 g/L $Na_2S \times 9H_2O$) in a 250 ml bottle were inoculated with 5 ml of a frozen cryoculture of *Clostridium kluyveri* and incubated at 37°C for 144 h to an $OD_{600nm}$ >0.3.

For the main culture 200 ml of fresh DMSZ52 medium in a 500 ml bottle were inoculated with centrifuged cells from the preculture to an $OD_{600nm}$ of 0.1. This growing culture was incubated at 37°C for 25 h to an $OD_{600nm}$ >0.4. Then the cell suspension was centrifuged, washed with production buffer (pH 6.16; 4.16 g/L K-acetate, 10.0 g/l ethanol) and centrifuged again.

For the production cultures, 200 ml of production buffer in a 500 ml bottle was inoculated with the washed cells from the main culture to an $OD_{600nm}$ of 0.2. In a first culture, at the beginning 1.0 g/l butyric acid was added to the production buffer, in a second culture, no butyric acid was added to the production buffer. The cultures were capped with a butyl rubber stopper and incubated for 71h at 37°C and 100 rpm in an open water shaking bath. At the start and end of the culturing period, samples were taken. These were tested for optical density, pH and the different analytes (tested by NMR).

The results showed that in the production phase of the butyric acid supplemented culture the amount of acetate decreased from 3.1 g/l to 1.1 g/l and the amount of ethanol decreased from 10.6 g/l to 7.5 g/l. Also, the concentration of butyric acid was increased from 1.2 g/l to 2.2 g/l and the concentration of hexanoic acid was increased from 0.04 g/l to 2.30 g/l.

In the production phase of the non-supplemented culture the amount of acetate decreased from 3.0 g/l to 1.3 g/l and the amount of ethanol decreased from 10.2 g/l to 8.2 g/l. Also, the concentration of butyric acid was increased from 0.1 g/l to 1.7 g/l and the concentration of hexanoic acid was increased from 0.01 g/l to 1.40 g/l.

**Example 3**

*Cultivation of Clostridium kluyveri in presence of decane and TOPO*

The bacterium *Clostridium kluyveri* was cultivated for the biotransformation of ethanol and acetate to hexanoic acid. For the *inSitu* extraction of the produced hexanoic acid a mixture of decane with trioctylphosphineoxide (TOPO) was added to the cultivation. All cultivation steps were carried out under anaerobic conditions in pressure-resistant glass bottles that can be closed airtight with a butyl rubber stopper.

For the preculture 250 ml of Veri01 medium (pH 7.0; 10 g/L potassium acetate, 0.31 g/L $K_2HPO_4$, 0.23 g/L $KH_2PO_4$, 0.25 g/L $NH_4Cl$, 0.20 g/L $MgSO_4 \times 7\ H_2O$, 10 µl /L HCl (7.7 M), 1.5 mg/L $FeCl_2 \times 4\ H_2O$, 36 µg/L $ZnCl_2$, 64 µg/L $MnCl_2 \times 4\ H_2O$, 6 µg/L $H_3BO_3$, 190 µg/L $CoCl_2 \times 6\ H_2O$, 1.2 µg/L $CuCl_2 \times 6\ H_2O$, 24 µg/L $NiCl_2 \times 6\ H_2O$, 36 µg/L $Na_2MO_4 \times 2\ H_2O$, 0.5 mg/L NaOH, 3 µg/L $Na_2SeO_3 \times 5\ H_2O$, 4 µg/L $Na_2WO_4 \times 2\ H_2O$, 100 µg/L vitamin B12, 80 µg/L p-aminobenzoic acid, 20 µg/L D(+) Biotin, 200 µg/L nicotinic acid, 100 µg/L D-Ca-pantothenate, 300 µg/L pyridoxine hydrochloride, 200 µg/l thiamine-HCl × $2H_2O$, 20 ml/L ethanol, 2.5 g/L NaHCOs, 65 mg/L glycine, 24 mg/L histidine, 64.6 mg/L isoleucine, 93.8 mg/L leucine, 103 mg/L lysine, 60.4 mg/L arginine, 21.64 mg/L L-cysteine-HCl, 21 mg/L methionine, 52 mg/L proline, 56.8 mg/L serine, 59 mg/L threonine, 75.8 mg/L valine) were inoculated with 10 ml of a living culture of *Clostridium kluyveri* to a start $OD_{600nm}$ of 0.1.

The cultivation was carried out in a 1000 mL pressure-resistant glass bottle at 37°C, 150 rpm and a ventilation rate of 1 L/h with 100% $CO_2$ in an open water bath shaker for 671 h. The gas was discharged into the headspace of the reactor. The pH was hold at 6.2 by automatic addition of 100 g/L NaOH solution. Fresh medium was continuously fed to the reactor with a dilution rate of 2.0 $d^{-1}$ and fermentation broth continuously removed from the reactor through a KrosFlo® hollow fibre polyethersulfone membrane with a pore size of 0.2 µm (Spectrumlabs, Rancho Dominguez, USA) to retain the cells in the reactor.

For the main culture 100 ml of fresh Veri01 medium in a 250 ml bottle was inoculated with centrifuged cells from the preculture to an $OD_{600nm}$ of 0.1. Additional 1 ml of a mixture of 6% (w/w) TOPO in decane was added. The culture was capped with a butyl rubber stopper and incubated at 37°C and 150 rpm in an open water bath shaker for 43 h under 100% $CO_2$ atmosphere.

During cultivation several 5 mL samples were taken to determinate $OD_{600nm}$, pH und product formation. The determination of the product concentrations was performed by semi-quantitative 1H-NMR spectroscopy. As an internal quantification standard sodium trimethylsilylpropionate (T(M)SP) was used.

During the main cultivation the concentration of butyrate increased from 0.14 g/L to 2.12 g/L and the concentration of hexanoate increased from 0.22 g/L to 0.91 g/L, whereas the concentration of ethanol decreased from 15.04 to 11.98 g/l and the concentration of acetate decreased from 6.01 to 4.23 g/L.

The $OD_{600nm}$ decreased during this time from 0.111 to 0.076.

**Example 4**

*Cultivation of Clostridium kluyveri in presence of tetradecane and TOPO*

The bacterium *Clostridium kluyveri* was cultivated for the biotransformation of ethanol and acetate to hexanoic acid. For the *inSitu* extraction of the produced hexanoic acid a mixture of tetradecane with trioctylphosphineoxide (TOPO) was added to the cultivation. All cultivation steps were carried out under anaerobic conditions in pressure-resistant glass bottles that can be closed airtight with a butyl rubber stopper.

The precultivation of *Clostridium kluyveri* was carried out in a 1000 mL pressure-resistant glass bottle in 250 ml of EvoDM24 medium (pH 5.5; 0.429 g/L Mg-acetate, 0.164 g/l Na-acetate, 0.016 g/L Ca-acetate, 2.454 g/l K-acetate, 0.107 mL/L $H_3PO_4$ (8.5%), 0.7 g/L $NH_4$acetate, 0.35 mg/L Co-acetate, 1.245 mg/L Ni-acetate, 20 µg/L d-biotin, 20 µg/L folic acid, 10 µg/L pyridoxine-HCl, 50 µg/L thiamine-HCl, 50 µg/L Riboflavin, 50 µg/L nicotinic acid, 50 µg/L Ca-pantothenate, 50 µg/L Vitamin B12, 50 µg/L p-aminobenzoate, 50 µg/L lipoic acid, 0.702 mg/L $(NH4)_2Fe(SO_4)_2 \times 4\ H_2O$, 1 ml/L KS-acetate (93,5 mM), 20 mL/L ethanol, 0.37 g/L acetic acid) at 37°C, 150 rpm and a ventilation rate of 1 L/h with a mixture of 25 % $CO_2$ and 75 % $N_2$ in an open water bath shake. The gas was discharged into the headspace of

the reactor. The pH was hold at 5.5 by automatic addition of 2.5 M $NH_3$ solution. Fresh medium was continuously feeded to the reactor with a dilution rate of 2.0 $d^{-1}$ and fermentation broth continuously removed from the reactor through a KrosFlo® hollow fibre polyethersulfone membrane with a pore size of 0.2 μm (Spectrumlabs, Rancho Dominguez, USA) to retain the cells in the reactor and hold an $OD_{600nm}$ of ~1.5.

For the main culture 100 ml of Veri01 medium (pH 6.5; 10 g/L potassium acetate, 0.31 g/L $K_2HPO_4$, 0.23 g/L $KH_2PO_4$, 0.25 g/L $NH_4Cl$, 0.20 g/L $MgSO_4 \times 7\ H_2O$, 10 μl /L HCl (7.7 M), 1.5 mg/L $FeCl_2 \times 4\ H_2O$, 36 μg/L $ZnCl_2$, 64 μg/L $MnCl_2 \times 4\ H_2O$, 6 μg/L $H_3BO_3$, 190 μg/L $CoCl_2 \times 6\ H_2O$, 1.2 μg/L $CuCl_2 \times 6\ H_2O$, 24 μg/L $NiCl_2 \times 6\ H_2O$, 36 μg/L $Na_2MO_4 \times 2\ H_2O$, 0.5 mg/L NaOH, 3 μg/L $Na_2SeO_3 \times 5\ H_2O$, 4 μg/L $Na_2WO_4 \times 2\ H_2O$, 100 μg/L vitamin B12, 80 μg/L p-aminobenzoic acid, 20 μg/L D(+) Biotin, 200 μg/L nicotinic acid, 100 μg/L D-Ca-pantothenate, 300 μg/L pyridoxine hydrochloride, 200 μg/l thiamine-HCl × $2H_2O$, 20 ml/L ethanol, 2.5 g/L NaHCOs, 65 mg/L glycine, 24 mg/L histidine, 64.6 mg/L isoleucine, 93.8 mg/L leucine, 103 mg/L lysine, 60.4 mg/L arginine, 21.64 mg/L L-cysteine-HCl, 21 mg/L methionine, 52 mg/L proline, 56.8 mg/L serine, 59 mg/L threonine, 75.8 mg/L valine, 2.5 mL/L HCL 25 %) in a 250 ml bottle were inoculated with centrifuged cells from the preculture to an $OD_{600nm}$ of 0.1. Additional 1 ml of a mixture of 6% (w/w) TOPO in tetradecane was added. The culture was capped with a butyl rubber stopper and incubated at 37°C and 150 rpm in an open water bath shaker for 47 h under 100% $CO_2$ atmosphere.

During cultivation several 5 mL samples were taken to determinate $OD_{600nm}$, pH und product formation. The determination of the product concentrations was performed by semiquantitative 1H-NMR spectroscopy. As an internal quantification standard sodium trimethylsilylpropionate (T(M)SP) was used.

During the main cultivation the concentration of butyrate increased from 0.05 g/L to 3.78 g/L and the concentration of hexanoate increased from 0.09 g/L to 4.93 g/L, whereas the concentration of ethanol decreased from 15.52 to 9.36 g/l and the concentration of acetate decreased from 6.36 to 2.49 g/L.

The $OD_{600nm}$ increased during this time from 0.095 to 0.685.

**Example 5**

*Cultivation of Clostridium kluyveri in presence of hexadecane and TOPO*

The bacterium *Clostridium kluyveri* was cultivated for the biotransformation of ethanol and acetate to hexanoic acid. For the *inSitu* extraction of the produced hexanoic acid a mixture of hexadecane with trioctylphosphineoxide (TOPO) was added to the cultivation. All cultivation steps were carried out under anaerobic conditions in pressure-resistant glass bottles that can be closed airtight with a butyl rubber stopper.

For the preculture 250 ml of Veri01 medium (pH 7.0; 10 g/L potassium acetate, 0.31 g/L $K_2HPO_4$, 0.23 g/L $KH_2PO_4$, 0.25 g/L $NH_4Cl$, 0.20 g/L $MgSO_4 \times 7\ H_2O$, 10 μl /L HCl (7.7 M), 1.5 mg/L $FeCl_2 \times 4\ H_2O$, 36 μg/L $ZnCl_2$, 64 μg/L $MnCl_2 \times 4\ H_2O$, 6 μg/L $H_3BO_3$, 190 μg/L $CoCl_2 \times 6\ H_2O$, 1.2 μg/L $CuCl_2 \times 6\ H_2O$, 24 μg/L $NiCl_2 \times 6\ H_2O$, 36 μg/L $Na_2MO_4 \times 2\ H_2O$, 0.5 mg/L NaOH, 3 μg/L $Na_2SeO_3 \times 5\ H_2O$, 4 μg/L $Na_2WO_4 \times 2\ H_2O$, 100 μg/L vitamin B12, 80 μg/L p-aminobenzoic acid, 20 μg/L D(+) Biotin, 200 μg/L nicotinic acid, 100 μg/L D-Ca-pantothenate, 300 μg/L pyridoxine hydrochloride, 200 μg/l thiamine-HCl × $2H_2O$, 20 ml/L ethanol, 2.5 g/L NaHCOs, 65 mg/L glycine, 24 mg/L histidine, 64.6 mg/L isoleucine, 93.8 mg/L leucine, 103 mg/L lysine, 60.4 mg/L arginine, 21.64 mg/L L-cysteine-HCl, 21 mg/L methionine, 52 mg/L proline, 56.8 mg/L serine, 59 mg/L threonine, 75.8 mg/L valine) were inoculated with 10 ml of a living culture of *Clostridium kluyveri* to a start $OD_{600nm}$ of 0.1.

The cultivation was carried out in a 1000 mL pressure-resistant glass bottle at 37°C, 150 rpm and a ventilation rate of 1 L/h with 100% $CO_2$ in an open water bath shaker for 671 h. The gas was discharged into the headspace of the reactor. The pH was hold at 6.2 by automatic addition of 100 g/L NaOH solution. Fresh medium was continuously fed to the reactor with a dilution rate of 2.0 $d^{-1}$ and fermentation broth continuously removed from the reactor through a KrosFlo® hollow fibre polyethersulfone membrane with a pore size of 0.2 μm (Spectrumlabs, Rancho Dominguez, USA) to retain the cells in the reactor.

For the main culture 100 ml of fresh Veri01 medium in a 250 ml bottle was inoculated with centrifuged cells from the preculture to an $OD_{600nm}$ of 0.1. Additional 1 ml of a mixture of 6% (w/w) TOPO in hexadecane was added. The culture was capped with a butyl rubber stopper and incubated at 37°C and 150 rpm in an open water bath shaker for 43 h under 100% $CO_2$ atmosphere.

During cultivation several 5 mL samples were taken to determinate $OD_{600nm}$, pH und product formation. The determination of the product concentrations was performed by semi-quantitative 1H-NMR spectroscopy. As an internal quantification standard sodium trimethylsilylpropionate (T(M)SP) was used.

During the main cultivation the concentration of butyrate increased from 0.14 g/L to 2.86 g/L and the concentration of hexanoate increased from 0.20 g/L to 2.37 g/L, whereas the concentration of ethanol decreased from 14.59 to 10.24 g/l and the concentration of acetate decreased from 5.87 to 3.32 g/L.

The $OD_{600nm}$ increased during this time from 0.091 to 0.256.

**Example 6**

*Cultivation of Clostridium kluyveri in presence of heptadecane and TOPO*

The bacterium *Clostridium kluyveri* was cultivated for the biotransformation of ethanol and acetate to hexanoic acid. For the *inSitu* extraction of the produced hexanoic acid a mixture of heptadecane with trioctylphosphineoxide (TOPO) was added to the cultivation. All cultivation steps were carried out under anaerobic conditions in pressure-resistant glass bottles that can be closed airtight with a butyl rubber stopper.

For the preculture 250 ml of Veri01 medium (pH 7.0; 10 g/L potassium acetate, 0.31 g/L $K_2HPO_4$, 0.23 g/L $KH_2PO_4$, 0.25 g/L $NH_4Cl$, 0.20 g/L $MgSO_4 \times 7\ H_2O$, 10 μl /L HCl (7.7 M), 1.5 mg/L $FeCl_2 \times 4\ H_2O$, 36 μg/L $ZnCl_2$, 64 μg/L $MnCl_2 \times 4\ H_2O$, 6 μg/L $H_3BO_3$, 190 μg/L $CoCl_2 \times 6\ H_2O$, 1.2 μg/L $CuCl_2$ X 6 $H_2O$, 24 μg/L $NiCl_2 \times 6\ H_2O$, 36 μg/L $Na_2MO_4 \times 2\ H_2O$, 0.5 mg/L NaOH, 3 μg/L $Na_2SeO_3 \times 5\ H_2O$, 4 μg/L $Na_2WO_4 \times 2\ H_2O$, 100 μg/L vitamin B12, 80 μg/L p-aminobenzoic acid, 20 μg/L D(+) Biotin, 200 μg/L nicotinic acid, 100 μg/L D-Ca-pantothenate, 300 μg/L pyridoxine hydrochloride, 200 μg/l thiamine-HCl $\times$ $2H_2O$, 20 ml/L ethanol, 2.5 g/L NaHCOs, 65 mg/L glycine, 24 mg/L histidine, 64.6 mg/L isoleucine, 93.8 mg/L leucine, 103 mg/L lysine, 60.4 mg/L arginine, 21.64 mg/L L-cysteine-HCl, 21 mg/L methionine, 52 mg/L proline, 56.8 mg/L serine, 59 mg/L threonine, 75.8 mg/L valine) were inoculated with 10 ml of a living culture of *Clostridium kluyveri* to a start $OD_{600nm}$ of 0.1.

The cultivation was carried out in a 1000 mL pressure-resistant glass bottle at 37°C, 150 rpm and a ventilation rate of 1 L/h with 100% $CO_2$ in an open water bath shaker for 671 h. The gas was discharged into the headspace of the reactor. The pH was hold at 6.2 by automatic addition of 100 g/L NaOH solution. Fresh medium was continuously feeded to the reactor with a dilution rate of 2.0 $d^{-1}$ and fermentation broth continuously removed from the reactor through a KrosFlo® hollow fibre polyethersulfone membrane with a pore size of 0.2 μm (Spectrumlabs, Rancho Dominguez, USA) to retain the cells in the reactor.

For the main culture 100 ml of fresh Veri01 medium in a 250 ml bottle were inoculated with centrifuged cells from the preculture to an $OD_{600nm}$ of 0.1. Additional 1 ml of a mixture of 6% (w/w) TOPO in heptadecane was added. The culture was capped with a butyl rubber stopper and incubated at 37°C and 150 rpm in an open water bath shaker for 43 h under 100% $CO_2$ atmosphere.

During cultivation several 5 mL samples were taken to determinate $OD_{600nm}$, pH und product formation. The determination of the product concentrations was performed by semiquantitative 1H-NMR spectroscopy. As an internal quantification standard sodium trimethylsilylpropionate (T(M)SP) was used.

During the main cultivation the concentration of butyrate increased from 0.15 g/L to 2.82 g/L and the concentration of hexanoate increased from 0.19 g/L to 2.85 g/L, whereas the concentration of ethanol decreased from 14.34 to 9.58 g/l and the concentration of acetate decreased from 5.88 to 3.20 g/L.

The $OD_{600nm}$ increased during this time from 0.083 to 0.363.

**Example 7**

*Cultivation of Clostridium kluyveri in presence of dodecane and TOPO*

The bacterium *Clostridium kluyveri* was cultivated for the biotransformation of ethanol and acetate to hexanoic acid. For the *inSitu* extraction of the produced hexanoic acid a mixture of dodecane with trioctylphosphineoxide (TOPO) was added to the cultivation. All cultivation steps were carried out under anaerobic conditions in pressure-resistant glass bottles that can be closed airtight with a butyl rubber stopper.

For the preculture 250 ml of Veri01 medium (pH 7.0; 10 g/L potassium acetate, 0.31 g/L $K_2HPO_4$, 0.23 g/L $KH_2PO_4$, 0.25 g/L $NH_4Cl$, 0.20 g/L $MgSO_4 \times 7\ H_2O$, 10 μl /L HCl (7.7 M), 1.5 mg/L $FeCl_2 \times 4\ H_2O$, 36 μg/L $ZnCl_2$, 64 μg/L $MnCl_2 \times 4\ H_2O$, 6 μg/L $H_3BO_3$, 190 μg/L $CoCl_2 \times 6\ H_2O$, 1.2 μg/L $CuCl_2 \times 6\ H_2O$, 24 μg/L $NiCl_2 \times 6\ H_2O$, 36 μg/L $Na_2MO_4 \times 2\ H_2O$, 0.5 mg/L NaOH, 3 μg/L $Na_2SeO_3 \times 5\ H_2O$, 4 μg/L $Na_2WO_4 \times 2\ H_2O$, 100 μg/L vitamin B12, 80 μg/L p-aminobenzoic acid, 20 μg/L D(+) Biotin, 200 μg/L nicotinic acid, 100 μg/L D-Ca-pantothenate, 300 μg/L pyridoxine hydrochloride, 200 μg/l thiamine-HCl $\times$ $2H_2O$, 20 ml/L ethanol, 2.5 g/L NaHCOs, 65 mg/L glycine, 24 mg/L histidine, 64.6 mg/L isoleucine, 93.8 mg/L leucine, 103 mg/L lysine, 60.4 mg/L arginine, 21.64 mg/L L-cysteine-HCl, 21 mg/L methionine, 52 mg/L proline, 56.8 mg/L serine, 59 mg/L threonine, 75.8 mg/L valine) were inoculated with 10 ml of a living culture of *Clostridium kluyveri* to a start $OD_{600nm}$ of 0.1.

The cultivation was carried out in a 1000 mL pressure-resistant glass bottle at 37°C, 150 rpm and a ventilation rate of 1 L/h with 100% $CO_2$ in an open water bath shaker for 671 h. The gas was discharged into the headspace of the reactor. The pH was hold at 6.2 by automatic addition of 100 g/L NaOH solution. Fresh medium was continuously feeded to the reactor with a dilution rate of 2.0 $d^{-1}$ and fermentation broth continuously removed from the reactor through a KrosFlo® hollow fibre polyethersulfone membrane with a pore size of 0.2 μm (Spectrumlabs, Rancho Dominguez, USA) to retain the cells in the reactor.

For the main culture 100 ml of fresh Veri01 medium in a 250 ml bottle were inoculated with centrifuged cells from the preculture to an $OD_{600nm}$ of 0.1. Additional 1 ml of a mixture of 6% (w/w) TOPO in dodecane was added. The culture was capped with a butyl rubber stopper and incubated at 37°C and 150 rpm in an open water bath shaker for 43 h under 100% $CO_2$ atmosphere. During cultivation several 5 mL samples were taken to determinate $OD_{600nm}$, pH und product formation. The determination of the product concentrations was performed by semiquantitative 1H-NMR spectroscopy. As an internal quantification standard sodium trimethylsilylpropionate (T(M)SP) was used.

During the main cultivation the concentration of butyrate increased from 0.14 g/L to 2.62 g/L and the concentration of hexanoate increased from 0.22 g/L to 2.05 g/L, whereas the concentration of ethanol decreased from 14.62 to 10.64 g/l and the concentration of acetate decreased from 5.92 to 3.54 g/L.

The $OD_{600nm}$ increased during this time from 0.091 to 0.259.

**Example 8**

*Determination of the distribution coefficient for hexanoic acid between water and a mixture of*

*hexadecane and TOPO*

During all stages of the experiment, samples from both phases were taken for determination of pH and concentration of hexanoic acid by high performance liquid chromatography (HPLC). 100 g of an aqueous solution of 5 g/kg hexanoic acid and 33 g of a mixture of 6% trioctylphosphinoxide (TOPO) in hexadecane were filled in a separatory funnel and mixed for 1 minute at 37°C. Then the funnel was placed in a tripod ring and the emulsion was left to stand to separate spontaneously. The pH of the aqueous phase was measured. Then 1M NaOH solution was added to the funnel and mixed. The step of separation and sampling was repeated until a pH of 6.2 in the aqueous phase was reached. Samples from both phases were taken for later analysis at this point. The aqueous phase could be analyzed directly by HPLC. For the analysis of the organic phase the diluted hexanoic acid was first re-extracted to water (pH 12.0 by addition of 1 M NaOH) and then analyzed by HPLC. The distribution coefficient $K_D$ of hexanoic acid in the system of water and 6% TOPO in hexadecane was calculated from the concentrations of hexanoic acid in both phases.

$$K(D) = \frac{c(Hex, organic\ phase)}{c\,(Hex, aqueous\ phase)}$$

The $K_D$ for hexanoic acid in the system of water and 6% TOPO in hexadecane at pH 6.2 was 4.7.

**Example 9**

*Determination of the distribution coefficient for hexanoic acid between water and a mixture of heptadecane and TOPO*

During all stages of the experiment, samples from both phases were taken for determination of pH and concentration of hexanoic acid by high performance liquid chromatography (HPLC). 100 g of an aqueous solution of 5 g/kg hexanoic acid and 33 g of a mixture of 6% trioctylphosphinoxide (TOPO) in heptadecane were filled in a separatory funnel and mixed for 1 minute at 37°C. Then the funnel was placed in a tripod ring and the emulsion was left to stand to separate spontaneously. The pH of the aqueous phase was measured. 1M NaOH solution was added to the funnel and mixed. The step of separation and sampling was repeated until a pH of 6.2 in the aqueous phase was reached. Samples from both phases were taken for later analysis at this point. The aqueous phase could be analyzed directly by HPLC. For the analysis of the organic phase the diluted hexanoic acid was first re-extracted to water (pH 12.0 by addition of 1 M NaOH) and then analyzed by HPLC. The distribution coefficient $K_D$ of hexanoic acid in the system of water and 6% TOPO in heptadecane was calculated from the concentrations of hexanoic acid in both phases.

$$K(D) = \frac{c(Hex, organic\ phase)}{c\,(Hex, aqueous\ phase)}$$

The $K_D$ for hexanoic acid in the system water and 6% TOPO in heptadecane at pH 6.2 was 5.0.

**Example 10**

*Determination of the distribution coefficient for hexanoic acid between water and a mixture of tetradecane and TOPO*

During all stages of the experiment, samples from both phases were taken for determination of pH and concentration of hexanoic acid by high performance liquid chromatography (HPLC). 130 g of an aqueous solution of 5 g/kg hexanoic acid plus 0.5 g/kg acetic acid and 15 g of a mixture of 6% trioctylphosphinoxid (TOPO) in tetradecane were filled in a separatory funnel and mixed for 1 minute at 37°C. Then the funnel was placed in a tripod ring and the emulsion was led stand to separate spontaneously. The pH of the aqueous phase was measured. 1M NaOH solution was added to the funnel and mixed. The step of separation and sampling was repeated until a pH of 6.2 in the aqueous phase was reached. Samples from both phases were taken for later analysis at this point. The aqueous phase could be analyzed directly by HPLC. For the analysis of the organic phase the diluted hexanoic acid was first re-extracted to water (pH 12.0 by addition of 1 M NaOH) and then analyzed by HPLC. The distribution coefficient $K_D$ of hexanoic acid in the system water and 6% TOPO in tetradecane was calculated from the concentrations of hexanoic acid in both phases.

$$K(D) = \frac{c(Hex, organic\ phase)}{c\ (Hex, aqueous\ phase)}$$

The $K_D$ for hexanoic acid in the system water and 6% TOPO in tetradecane at pH 6.9 was 1.3.

**Example 11**

*Cultivation of Clostridium kluyveri and Extraction of hexanoic acid*

The bacterium *Clostridium kluyveri* was cultivated for the biotransformation of ethanol and acetate to hexanoic acid. For the *inSitu* extraction of the produced hexanoic acid a mixture of tetradecane with trioctylphosphineoxide (TOPO) was continuously passed through the cultivation. All cultivation steps were carried out under anaerobic conditions in pressure-resistant glass bottles that can be closed airtight with a butyl rubber stopper.

The precultivation of *Clostridium kluyveri* was carried out in a 1000 mL pressure-resistant glass bottle in 250 ml of EvoDM45 medium (pH 5.5; 0.004 g/L Mg-acetate, 0.164 g/l Na-acetate, 0.016 g/L Ca-acetate, 0.25 g/l K-acetate, 0.107 mL/L $H_3PO_4$ (8.5%), 2.92 g/L $NH_4$acetate, 0.35 mg/L Co-acetate, 1.245 mg/L Ni-acetate, 20 $\mu$g/L d-biotin, 20 $\mu$g/L folic acid, 10 $\mu$g/L pyridoxine-HCl, 50 $\mu$g/L thiamine-HCl, 50 $\mu$g/L Riboflavin, 50 $\mu$g/L nicotinic acid, 50 $\mu$g/L Ca-pantothenate, 50 $\mu$g/L Vitamin B12, 50 $\mu$g/L p-aminobenzoate, 50 $\mu$g/L lipoic acid, 0.702 mg/L $(NH4)_2Fe(SO_4)_2 \times 4\ H_2O$, 1 ml/L KS-acetate (93,5 mM), 20 mL/L ethanol, 0.37 g/L acetic acid) at 37°C, 150 rpm and a ventilation rate of 1 L/h with a mixture of 25 % $CO_2$ and 75 % $N_2$ in an open water bath shaker. The gas was discharged into the headspace of the reactor. The pH was hold at 5.5 by automatic addition of 2.5 M $NH_3$ solution. Fresh medium was continuously feeded to the reactor with a dilution rate of 2.0 $d^{-1}$ and fermentation broth continuously removed from the reactor through a KrosFlo® hollow fibre polyethersulfone membrane with a pore size of 0.2 $\mu$m (Spectrumlabs, Rancho Dominguez, USA) to retain the cells in the reactor and hold an $OD_{600nm}$ of ~1.5.

For the main culture 150 ml of EvoDM39 medium (pH 5.8; 0.429 g/L Mg-acetate, 0.164 g/l Na-acetate, 0.016 g/L Ca-acetate, 2.454 g/l K-acetate, 0.107 mL/L $H_3PO_4$ (8.5%), 1.01 mL/L acetic acid, 0.35 mg/L Co-acetate, 1.245 mg/L Ni-acetate, 20 $\mu$g/L d-biotin, 20 $\mu$g/L folic acid, 10 $\mu$g/L pyridoxine-HCl, 50 $\mu$g/L thiamine-HCl, 50 $\mu$g/L Riboflavin, 50 $\mu$g/L nicotinic acid, 50 $\mu$g/L Ca-pantothenate, 50 $\mu$g/L Vitamin B12, 50 $\mu$g/L p-aminobenzoate, 50 $\mu$g/L lipoic acid, 0.702 mg/L $(NH4)_2Fe(SO_4)_2 \times 4\ H_2O$, 1 ml/L KS-acetate (93,5 mM), 20 mL/L ethanol, 8.8 mL $NH_3$ solution (2,5 mol/L), 27.75 ml/L acetic acid (144 g/L))

in a 1000 ml bottle were inoculated with 100 ml cell broth from the preculture to an $OD_{600nm}$ of 0.71.

The cultivation was carried out at 37°C, 150 rpm and a ventilation rate of 1 L/h with a mixture of 25 % $CO_2$ and 75 % $N_2$ in an open water bath shaker for 65 h. The gas was discharged into the headspace of the reactor. The pH was hold at 5.8 by automatic addition of 2.5 M $NH_3$ solution. Fresh medium was continuously feeded to the reactor with a dilution rate of 0.5 $d^{-1}$ and fermentation broth continuously removed from the reactor by holding an $OD_{600nm}$ of ~0.5. Additional 120 g of a mixture of 6% (w/w) TOPO in tetradecane was added to the fermentation broth. Then this organic mixture was continuously feeded to the reactor and the organic phase also continuously removed from the reactor with a dilution rate of 1 $d^{-1}$.

During cultivation several 5 mL samples from both, the aqueous and the organic phase, were taken to determinate $OD_{600nm}$, pH und product formation. The determination of the product concentrations was performed by semi-quantitative 1H-NMR spectroscopy. As an internal quantification standard sodium trimethylsilylpropionate (T(M)SP) was used.

During the main cultivation in the aqueous phase a steady state concentration of 8.18 g/L ethanol, 3.20 g/L acetate, 1.81 g/L butyrate and 0.81 g/L hexanoate was reached. The $OD_{600nm}$ remained stable at 0.5. In the organic phase a steady state concentration of 0.43 g/kg ethanol, 0.08 g/kg acetate, 1.13 g/kg butyrate and 8.09 g/kg hexanoate was reached. After the experiment the cells remained viable while transferred to further cultivations.

The distribution coefficient $K_D$ of the substrates and products in the system aqueous medium and 6% TOPO in tetradecane was calculated from the concentrations in both phases.

$$K(D) = \frac{c(organic\ phase)}{c\ (aqueous\ phase)}$$

The $K_D$ in the steady state was 0.05 for ethanol, 0.03 for acetic acid, 0.62 for butyric acid and 9.99 for hexanoic acid.

**Example 12**

*Ketonization of hexanoic acid*

The ketonization was conducted in a heated continuous flow-bed reactor. At first, the reactor was charged with magnesium oxide on silica (50 wt.%, 14.00 g) and heated under an argon flow (54 mL/min) at 330 °C for one hour. The temperature was raised to 360 °C. Than a mixture of hexanoic acid in tetradecane (v/v: 3/1) was continuously fed to the reactor with a rate of 3.3 mL/h. The gaseous out stream was collected by two cooling traps, which were cooled with water and a mixture of dry ice and isopropanol. The collected fractions were weighted and analyzed by gas chromatography (GC) for their composition. In total, 370.65 g of hexanoic acid was fed to the reactor, which equals to a maximum theoretical yield of 271.70 g of 6-undecanone and 28.75 g of water and 70.21 g of carbon dioxide as by-products. The obtained amount of 6-undecanone was 267.67 g and the amount of water was 28.32 g. This corresponds to a 99% mass recovery at full conversion. The high productivity and selectivity were confirmed by regular GC measurements, as only traces of hexanoic acid and no side-products were detected.

**Example 13**

*Cross ketonization of hexanoic acid with palmitic acid.*

The technical procedure of the cross ketonization is identical to the sole ketonization of hexanoic acid (example 12) except the composition of the substrate feed. The feed consists of hexanoic acid (116.16 g, 1.00 mol) and palmitic acid (256.43 g, 1.00 mol) as substrates and tetradecane (124.20 g) as internal standard. The substrate feed is added with a rate of 3.3 mL/h and reacted at a temperature of 360 °C. The presence of two alkanoic acids leads two a product mixture of three ketones: 6-undecanone, 6-henicosanone and 16-hentriacontanone. At full conversion, the amounts obtained are 42.58 g of 6-undecanone, 155.29 g of 6-henicosanone and 112.71 g of 16-hentriacontanone.

**Example 14**

*Cross ketonization of hexanoic acid with acetic acid.*

The technical procedure of the cross ketonization is identical to the sole ketonization of hexanoic acid (example 12) except the composition of the substrate feed. The feed consists of hexanoic acid 232.32 g, 2.00 mol) and acetic acid (120.10 g, 2.00 mol) as substrates and tetradecane (117.47 g) as internal standard. The substrate feed is added with a rate of 3.3 mL/h and reacted at a temperature of 360 °C. The presence of two alkanoic acids leads two a product mixture of three ketones: 2-propanone, 2-heptanone and 6-undecanone. At full conversion, the amounts obtained are 29.04 g of 2-propanone, 114.19 g of 2-heptanone and 85.15 g of 6-undecanone.

**Example 15**

*Hydrogenation of the higher alkanone to a corresponding higher secondary alkanol*

The hydrogenation reactions for $C_{11}$ ketone (6-undecanone) to alcohol (6-undecanol) were performed in a 300 ml autoclave reactor (PARR Instrument Company). The reactor was placed in an aluminum block and the temperature was controlled by a thermocouple placed inside the reactor. Typically, 30 mg of solid catalyst, 170.3 mg, 1.0 mmol of

substrate was added to a 4 ml glass vial having an oven dried magnetic stirrer. 2.0 ml of dry Toluene was used as solvent, vial was fitted with a screw cap and a needle was inserted through the septum. (The vial is placed in the reactor.) The reactor was purged three times with 10 bar of $H_2$ and then the pressure was increased to 20 bar. The reactor was heated to the desired temperature (120°C) for 20 h. After the reaction, the reactor was cooled down to 5°C using an ice bath, the gas phase was slowly released and the remaining liquid was carefully separated from the solid catalyst and was analyzed separately using an internal standard (100 μL n-hexadecane).

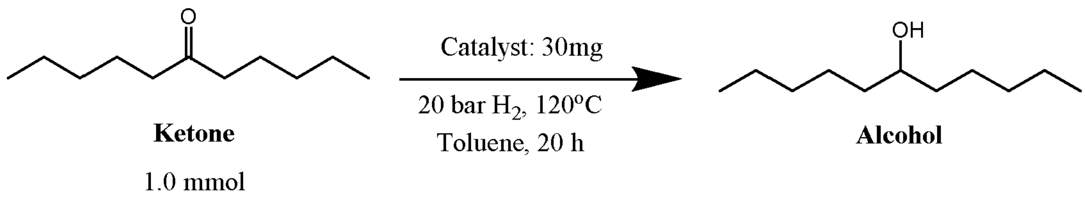


The ketone hydrogenation was investigated on different supported heterogeneous catalysts. As shown in the Table below, Zeolite based catalysts which showed excellent alcohol dehydration activity to olefin were not active in hydrogenation reaction of ketone (entry 1-3). Ru based catalyst (entry 4) achieved nearly full conversion and alcohol yield of 97%. Although Carbon supported Cobalt catalyst in entry 5 was also not active but to our surprise, changing the type of support material significantly improved the activity with $SiO_2$ based catalyst showing more than 80% ketone conversion. The most optimum catalyst (entry 7) i-e 3.0Co@γ-$Al_2O_3$ showed 99% ketone conversion and an improved alcohol yield of 98%.

| Entry | Catalyst | Ketone Conversion (%) | Alcohol GC-yield (mol %) |
|---|---|---|---|
| 1 | 0.5 Pd@ZSM-5 | 0 | 0 |
| 2 | 5.0 Ni@ZSM-5 | 0 | 0 |
| 3 | 5.0 Mo@ZSM-5 | 0 | 0 |
| 4 | 3.0 Ru-Phen@C | 99 | 97 |
| 5 | 3.0 Co-Phen@C | 0 | 0 |
| 6 | 3.0 Co@$SiO_2$ | 85 | 80 |
| **7** | **3.0 Co@γ-$Al_2O_3$** | **99** | **98** |

The catalyst preparation method is as follows:
**RR-168:** 3wt%Co@γ-$Al_2O_3$, using Ascorbic acid as reductant and glucose as capping agent in $H_2O$, Pyrolysis at 800°C for 2 h, Co salt is Cobalt(II) nitrate hexahydrate. In a typical synthesis, 149 mg, 0.51 mmol of $Co(NO_3)_2$. $6H_2O$ was dissolved in 20 ml D.I $H_2O$ followed by the stepwise addition of aqueous solutions of 265 mg, 3.0 mmol Ascorbic acid and 92 mg, 1 mmol of D-(+)-Glucose. The contents were stirred at 90°C for 2-3 h. Next, 1.0 g of γ-$Al_2O_3$ support was added and the slurry was stirred overnight at R.T. Excess water was removed through centrifugation and the solids were dried in oven at 120°C for 10 h and then pyrolyzed at 800°C for 2 h under argon atmosphere.

**Example 16**

***Alcohol dehydration to olefin***

**Example 16.1**

*Catalyst preparation:*

The dehydration step was performed over an acidic heterogeneous catalyst. Different materials were used to optimize the olefin yield and selectivity. Initially blank acidic supports such as Amberlyst-15, γ-$Al_2O_3$, H-ZSM-5 were investigated. Several active metals including: Pd, Ni, Co, Fe, Mo and Mn were also incorporated. Among the many tested samples, RR-111 (5wt%Mo@H-ZSM-5) was found to be the most active catalyst. The catalyst preparation method is as follows:
Using the oxalate route (oxalic acid as reductant) in $H_2O$ and pyrolysis at 600 °C for 2 h. Mo salt is Ammonium molybdate tetrahydrate. In a typical synthesis, 643 mg, 0.52 mmol of $(NH_4)_6Mo_7O_{24} \times 4\ H_2O$ together with 90.03 mg, 1.0 mmol of

oxalic acid were dissolved in 30 ml deionized $H_2O$ and stirred at 60 °C for 1 h. Then, 1.0 g of H-ZSM-5 (Si/Al = 30), pre-calcined in Air at 450 °C for 4 h was added and the mixture was stirred at 30 °C overnight followed by drying at 105 °C for 8 h and pyrolysis under Argon atmosphere at 600 °C for 2 h.

**Example 16.2**

*Dehydration of olefin:*

The catalytic activity tests for the dehydration of sec. alcohol were initially performed in a 300 ml autoclave reactor (PARR Instrument Company). The reactor was placed in an aluminum block and the temperature was controlled by a thermocouple placed inside the reactor. Typically, 172 mg, 1.0 mmol of substrate was added to a 4 ml glass vial together with 2.0 ml of Toluene as solvent and 30 mg of solid catalyst. The reactor was purged three times with $N_2$ and then the pressure was increased to 20 bar. The reactor was heated to the desired temperature for 20 h. After the reaction, the reactor was cooled down to 5° C using an ice bath and the remaining liquid phase was carefully separated from the solid catalyst and was analyzed separately using an internal standard.

**Example 16.3**

*Heterogeneous Catalyzed Dehydration of 6-Undecanol to 5-Undecene:*

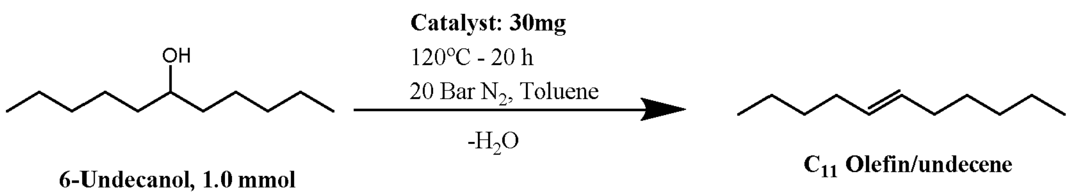


6-Undecanol, 1.0 mmol

$C_{11}$ Olefin/undecene

**Scheme 1** Dehydration of 6-UDA to corresponding Olefin using heterogeneous catalyst in batch reactor.

Catalyst screening experiments for dehydration of 6-UDA are shown in Table 1. Zeolite support (H-ZSM-5, Si/Al:30) showed superior catalytic activity as compared to other solid acid materials such as Amberlyst-15 and $\gamma$-$Al_2O_3$. The activity was further enhanced by doping Zeolite carrier with an active metal i-e Mo (table 1 entry 8). However, other metals such as Pd, Ni, Co showed a significant decrease in activity in comparison to blank H-ZSM-5. Toluene was found to be better solvent for this reaction and more than 90% olefin yield was obtained at 120° C (table 1 entry 9).

Table 1: Catalyst screening for Dehydration of 6-UDA to Olefin.

| Entry | Catalyst | Temp. | Solvent | Olefin GC-yield (mol %) |
|---|---|---|---|---|
| 1 | Amberlyst-15 | 140 | Toluene | 80 |
| 2 | H-ZSM-5 | 140 | Toluene | 83 |
| 3 | 0.5Pd@H-ZSM-5 | 140 | Toluene | 56 |
| 4 | 5.0Ni@H-ZSM-5 | 140 | Toluene | 50 |
| 5 | 3.0Co@H-ZSM-5 | 140 | Toluene | 0 |
| 6 | 0.5Pd@$\gamma$-$Al_2O_3$ | 140 | Toluene | 42 |
| 7 | 5.0Fe@H-ZSM-5 | 140 | Toluene | 80 |
| 8 | 5.0Mo@H-ZSM-5 | 140 | Toluene | 97 |
| 9 | 5.0Mo@H-ZSM-5 | 120 | Toluene | 93 |
| 10 | 5.0Mo@H-ZSM-5 | 120 | THF | 87 |

### Example 16.4

*Heterogeneous Catalyzed Dehydration of 2-Octanol to 2-Octene (isomeric mixture)*

To further demonstrate the concept of dehydration and subsequent carbonylation reactions, commercially relevant sec. alcohol i-e 2-octanol was selected. The optimum heterogeneous 5.0 Mo@H-ZSM-5 catalyst was used to successfully obtain an isomerized mixture of 2-4-octene. Similar reaction conditions were applied as in case of 6-undecanol and the catalyst achieved excellent yield for $C_8$ alkene (Scheme 2)

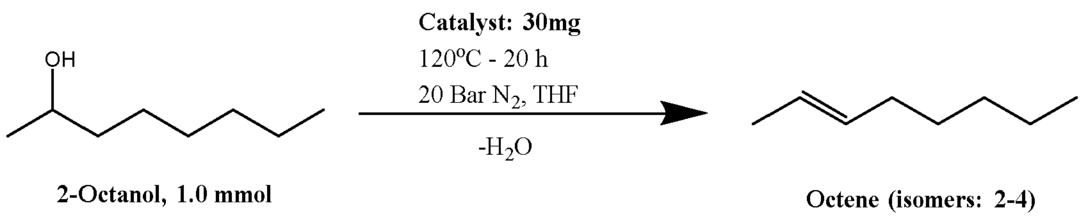


**Scheme 2:** Dehydration of 2-octanol to $C_8$ olefin.

| Entry | Catalyst | Temp. | Solvent | Olefin GC-yield (mol %) |
|---|---|---|---|---|
| 1 | 5.0Mo@H-ZSM-5 | 120 | Toluene | 99 |
| 2 | 5.0Mo@H-ZSM-5 | 120 | THF | 94 |

### Example 17

***Hydroxy-carbonylation of olefins***

*General procedure:*

A 4 mL screw-cap vial was charged with Palladium salts (1.0 mol% in terms of Pd atom), ligand (4.0 mol%), Acid (15 mol% in terms of H atom), 5-undecene (1.0 mmol, 0.2 ml) and an oven-dried stirring bar. The vial was closed by PTFE/white rubber septum (Wheaton 13 mm Septa) and phenolic cap and connected with Argon atmosphere with a needle. Then, the vial was evacuated under vacuum and recharged with argon for three times. After this, acetic acid (AcOH) (1.5 ml) and $H_2O$ (0.5 ml) were injected by syringe; the vial was fixed in an alloy plate and put into Paar 4560 series autoclave (300 mL) under argon atmosphere. At room temperature, the autoclave is flushed with carbon monoxide for three times and carbon monoxide was charged to 40 *bar.* The reaction was heated under specified temperature for 20 hours (stirring 1000 rpm). Afterwards, the autoclave was cooled to room temperature and the pressure was carefully released. Isooctane (100 μL) was added into the reaction as internal standard. A sample of the mixture was analyzed by gas chromatography.

### Example 17.1

*Hydroxy-carbonylation of 5-undecene to form dodecanoic acid*

As shown in Scheme 3, the $B_u$Pox-analog ligand **py$^t$bpx** (2,2'-[1,2-Phenylenebis[methylene[(1,1-dimethylethyl)phosphinidene]]]bis[pyridine], CAS: 2093415-45-1) gave excellent carbonylation yield in the presence of AcOH/$H_2O$ to give the corresponding dodecanoic acid.

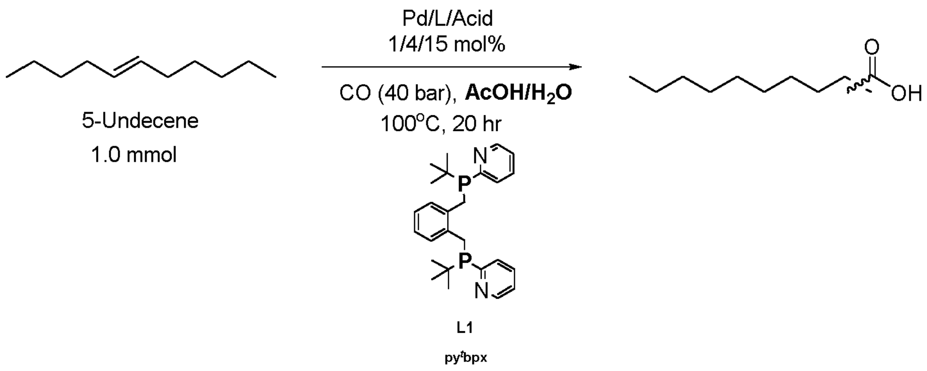


| Entry | Pd-Ligand | Acid | Yield (%) | Sel. % (l:b) |
|---|---|---|---|---|
| 1 | $Pd(acac)_2$-**L1** | PTSA | >99 | 62/38 |
| 2 | $Pd(dba)_2$-**L1** | PTSA | >99 | 60/40 |

**Scheme 3:** Hydroxy-Carbonylation of 5-undecene to $C_{12}$ Acid.

**Example 17.2**

*Hydroxy carbonylation of 2-octene to form nonanoic acid*

The hydroxy carbonylation of 2-octene (isomeric mixture) can be performed under the same reaction conditions as for 5-undecene. The active ligand (pytbpx) with $Pd(acac)_2$ and PTSA is expected to show superior acid yield (≥90%) and regioselectivity (n/iso:70/30).

**Example 18 (comparative)**

*Direct hydroxy-carbonylation of a secondary alcohol*

**Example 18.1**

Direct hydroxy-carbonylation of 6-Undecanol was investigated using the Pd/Lpytbpx/Acid catalyst system. The results indicate no carbonylation activity under the reaction conditions (Scheme 4). It became clear that the formation of alkene is the limiting step for such a transformation and esterification occurs as a side reaction.

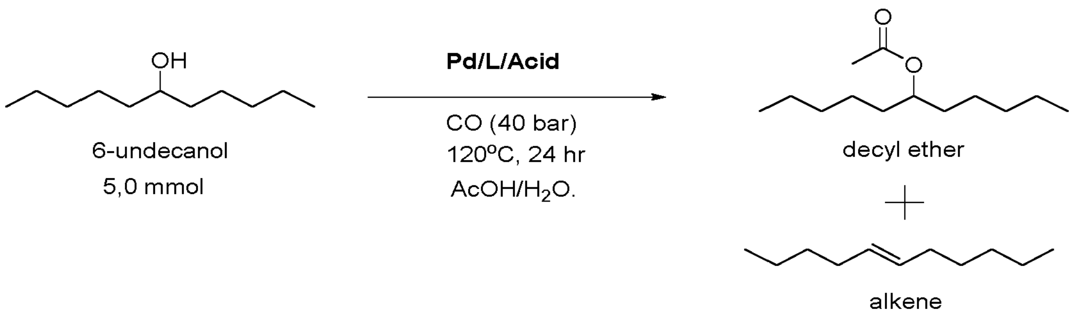


**Scheme 4** Direct carbonylation of a *sec.* alcohol using homogeneous Pd catalyst.

## Claims

1. A method of producing linear fatty acids comprising 7 to 28 carbon atoms or esters thereof, from ethanol and/or a linear alkanoic acid comprising 2 to 5 carbon atoms, the method comprising

(a) contacting ethanol and/or the linear alkanoic acid comprising 2 to 5 carbon atoms or any salts thereof with at least one microorganism capable of carrying out two-carbon chain elongation to produce as an intermediate a linear alkanoic acid comprising 4 to 7 carbon atoms and/or a salt thereof and/or an ester thereof;
(b) extracting the intermediate, its salt and/or ester thereof from (a) using at least one extractant, wherein the extractant comprises at least one alkyl-phosphine oxide and optionally at least one alkane comprising at least 12 carbon atoms; or at least one trialkylamine and at least one alkane comprising at least 12 carbon atoms;
(c) contacting the extracted intermediate linear alkanoic acid comprising 4 to 7 carbon atoms, its salt and/or ester thereof from (b) with at least one ketonization catalyst and optionally a further alkanoic acid comprising 2 to 22 carbon atoms under suitable reaction conditions for chemical ketonization of the intermediate linear alkanoic acid comprising 4 to 7 carbon atoms, its salt and/or ester thereof to a linear alkanone comprising 7 to 28 carbon atoms;
(d) contacting the linear alkanone comprising 7 to 28 carbon atoms from step (c) with at least one hydrogenation metal catalyst for catalytic hydrogenation of the linear alkanone comprising 7 to 28 carbon atoms to a corresponding secondary linear alkanol comprising 7 to 28 carbon atoms;
(e) dehydration of the secondary linear alkanol comprising 7 to 28 carbon atoms in the presence of an acidic heterogeneous catalyst to form a corresponding linear alkene comprising 7 to 28 carbon atoms;
(f) hydroxy or alkoxy carbonylation of the linear alkene comprising 7 to 28 carbon atoms to a linear fatty acid comprising 7 to 28 carbon atoms or an ester thereof in the presence of carbon monoxide, of an acid and of a catalyst comprising a transition metal.

**2.** The method according to claim 1, wherein the ketonization catalyst of (c) is a metal oxide catalyst or mixtures thereof.

**3.** The method according to claim 2, wherein the metal oxide catalyst or mixtures thereof is selected from the group consisting of heteropoly acid ($H_3PW_{12}O_{40}$) catalyst, titanium oxide ($TiO_2$) catalyst, cerium oxide ($CeO_2$) catalyst, zinc-chromium (Zn-Cr) mixed oxide catalyst, manganese oxide ($MnO_2$) catalyst, lanthanum oxide ($La_2O_3$) catalyst, magnesium oxide (MgO) catalyst, iron oxide (FeO, $FeO_2$, $Fe_2O_3$, $Fe_3O_4$, $Fe_4O_5$, $Fe_5O_6$, $Fe_5O_7$), , silicon-aluminium (Si-Al) mixed oxide catalyst and zirconia ($ZrO_2$) catalyst.

**4.** The method according to any one of the preceding claims, wherein the ketonization catalyst in step (c) is a zirconia aerogel catalyst.

**5.** The method according to any one of the preceding claims, wherein the suitable reaction conditions of step (c) comprises reaction temperatures of 150°C - 350 °C.

**6.** The method according to any one of the preceding claims, wherein the microorganism in (a) is selected from the group consisting of *Clostridium carboxidivorans* and *Clostridium kluyveri.*

**7.** The method according to any one of the preceding claims, wherein the alkyl-phosphine oxide is selected from the group consisting of trioctylphosphine oxide, hexylphosphine oxide, octylphosphine oxide and mixtures thereof and the alkane is selected from the group consisting of pentadecane, hexadecane, heptadecane, octadecane, and tetradecane.

**8.** The method according to any one of the preceding claims, wherein the alkyl-phosphine oxide is Trioctylphosphine oxide (TOPO) and the alkane is tetradecane.

**9.** The method according to claim 8, wherein the weight ratio of TOPO to tetradecane is between 1:100 to 1:10.

**10.** The method according to any one of the preceding claims, wherein the pH of the aqueous medium in (b) is maintained between 5.5 and 8.

**11.** The method according to any one of the preceding claims, wherein the hydrogenation metal catalyst of step (d) is selected from the group consisting of ruthenium (Ru) catalyst, rhenium (Re) catalyst, nickel (Ni) catalyst, iron (Fe), cobalt (Co) and platinum (Pt) catalyst.

**12.** The method according to any one of the preceding claims, wherein the acidic heterogeneous catalyst of step (e) is a catalyst comprising an aluminosilicate zeolite.

**13.** The method according of claim 12, wherein the acidic heterogeneous catalyst of step (e) comprises ZSM-5.

**14.** The method according to any one of the preceding claims, wherein the catalyst of step (e) further comprises a transition metal.

**15.** The method of claim 14, wherein the transition metal is molybdenum.

**16.** The method according to any one of the preceding claims, wherein the transition metal in the catalyst of step (f) has phosphine ligands.

**17.** The method of claim 16, wherein the phosphine ligand of the transition metal is (2,2'-[1,2-Phenylenebis[methylene[(1,1-dimethylethyl)phosphinidene]]]bis[pyridine].

**18.** The method according to any one of the preceding claims, wherein the transition metal in the catalyst of step (f) is palladium.

**19.** The method according to any one of the preceding claims, wherein the acid in step (f) is acetic acid.

**20.** The method according to any one of claims 1 to 18, wherein the acid in step (f) is p-toluene sulfonic acid.

**21.** The method of any of the preceding claims, wherein the linear alkanoic acid comprising 2 to 5 carbon atoms is acetic acid and the intermediate a linear alkanoic acid comprising 4 to 7 carbon atoms in steps (a) to (c) is hexanoic acid or an ester thereof.

**22.** The method of claim 21, wherein the linear alkanone comprising 7 to 28 carbon atoms steps (c) and (d) is 6-undecanone, the corresponding secondary linear alkanol comprising 7 to 28 carbon atoms in steps (d) and (e) is 6-undecanol, the linear alkene comprising 7 to 28 carbon atoms in steps (e) and (f) is 5-undecene and wherein the linear fatty acid comprising 7 to 28 carbon atoms is dodecanoic acid.

**Patentansprüche**

**1.** Verfahren zur Herstellung von linearen Fettsäuren, die 7 bis 28 Kohlenstoffatome umfassen, oder Estern davon aus Ethanol und/oder einer linearen Alkansäure, die 2 bis 5 Kohlenstoffatome umfasst, wobei das Verfahren umfasst:

(a) Inkontaktbringen von Ethanol und/oder der linearen Alkansäure, die 2 bis 5 Kohlenstoffatome umfasst, oder von beliebigen Salzen davon mit wenigstens einem Mikroorganismus, der fähig ist, eine Kettenverlängerung um zwei Kohlenstoffatome durchzuführen, um als Zwischenprodukt eine lineare Alkansäure, die 4 bis 7 Kohlenstoffatome umfasst, und/oder ein Salz davon und/oder einen Ester davon zu erzeugen;
(b) Extrahieren des Zwischenprodukts, seines Salzes und/oder Esters davon aus (a) unter Verwendung wenigstens eines Extraktionsmittels, wobei das Extraktionsmittel wenigstens ein Alkylphosphinoxid und gegebenenfalls wenigstens ein Alkan, das wenigstens 12 Kohlenstoffatome umfasst; oder wenigstens ein Trialkylamin und wenigstens ein Alkan, das wenigstens 12 Kohlenstoffatome umfasst, umfasst;
(c) Inkontaktbringen der extrahierten linearen Zwischenprodukt-Alkansäure, die 4 bis 7 Kohlenstoffatome umfasst, ihres Salzes und/oder Esters davon aus (b) mit wenigstens einem Ketonisierungskatalysator und gegebenenfalls einer weiteren Alkansäure, die 2 bis 22 Kohlenstoffatome umfasst, unter geeigneten Reaktionsbedingungen zur chemischen Ketonisierung der linearen Zwischenprodukt-Alkansäure, die 4 bis 7 Kohlenstoffatome umfasst, ihres Salzes und/oder Esters davon zu einem linearen Alkanon, das 7 bis 28 Kohlenstoffatome umfasst;
(d) Inkontaktbringen des linearen Alkanons, das 7 bis 28 Kohlenstoffatome umfasst, aus Schritt (c) mit wenigstens einem Hydrierungs-Metallkatalysator zur katalytischen Hydrierung des linearen Alkanons, das 7 bis 28 Kohlenstoffatome umfasst, zu einem entsprechenden sekundären linearen Alkanol, das 7 bis 28 Kohlenstoffatome umfasst;
(e) Dehydrieren des sekundären linearen Alkanols, das 7 bis 28 Kohlenstoffatome umfasst, in Gegenwart eines sauren heterogenen Katalysators, um ein entsprechendes lineares Alken, das 7 bis 28 Kohlenstoffatome umfasst, zu bilden;
(f) Hydroxy- oder Alkoxycarbonylierung des linearen Alkens, das 7 bis 28 Kohlenstoffatome umfasst, zu einer linearen Fettsäure, die 7 bis 28 Kohlenstoffatome umfasst, oder einem Ester davon in Gegenwart von Kohlenmonoxid, einer Säure und eines Katalysators, der ein Übergangsmetall umfasst.

2. Verfahren gemäß Anspruch 1, wobei der Ketonisierungskatalysator von (c) ein Metalloxidkatalysator oder Gemische davon ist.

3. Verfahren gemäß Anspruch 2, wobei der Metalloxidkatalysator oder Gemische davon ausgewählt ist aus der Gruppe bestehend aus Heteropolysäure ($H_3PW_{12}O_{40}$)-Katalysator, Titanoxid($TiO_2$)-Katalysator, Ceroxid($CeO_2$)-Katalysator, Zink-Chrom(Zn-Cr)-Mischoxidkatalysator, Manganoxid ($MnO_2$)-Katalysator, Lanthanoxid ($La_2O_3$)-Katalysator, Magnesiumoxid(MgO)-Katalysator, Eisenoxid (FeO, $FeO_2$, $Fe_2O_3$, $Fe_3O_4$, $Fe_4O_5$, FesOe, $Fe_5O_7$), Silicium-Aluminium(Si-Al)-Mischoxidkatalysator und Zirkoniumdioxid($ZrO_2$)-Katalysator.

4. Verfahren gemäß einem der vorstehenden Ansprüche, wobei der Ketonisierungskatalysator bei Schritt (c) ein Zirkoniumdioxid-Aerogel-Katalysator ist.

5. Verfahren gemäß einem der vorstehenden Ansprüche, wobei die geeigneten Reaktionsbedingungen von Schritt (c) Reaktionstemperaturen von 150°C bis 350 °C umfassen.

6. Verfahren gemäß einem der vorstehenden Ansprüche, wobei der Mikroorganismus bei (a) ausgewählt ist aus der Gruppe bestehend *ausClostridium carboxidivorans* und *Clostridium kluyveri.*

7. Verfahren gemäß einem der vorstehenden Ansprüche, wobei das Alkylphosphinoxid ausgewählt ist aus der Gruppe bestehend aus Trioctylphosphinoxid, Hexylphosphinoxid, Octylphosphinoxid und Gemischen davon und das Alkan ausgewählt ist aus der Gruppe bestehend aus Pentadecan, Hexadecan, Heptadecan, Octadecan und Tetradecan.

8. Verfahren gemäß einem der vorstehenden Ansprüche, wobei das Alkylphosphinoxid Trioctylphosphinoxid (TOPO) ist und das Alkan Tetradecan ist.

9. Verfahren gemäß Anspruch 8, wobei das Gewichtsverhältnis von TOPO zu Tetradecan zwischen 1:100 und 1:10 beträgt.

10. Verfahren gemäß einem der vorhergehenden Ansprüche, wobei der pH-Wert des wässrigen Mediums bei (b) zwischen 5,5 und 8 gehalten wird.

11. Verfahren gemäß einem der vorstehenden Ansprüche, wobei der Hydrierungs-Metallkatalysator von Schritt (d) ausgewählt ist aus der Gruppe bestehend aus Ruthenium(Ru)-Katalysator, Rhenium(Re)-Katalysator, Nickel(Ni)-Katalysator, Eisen-(Fe), Cobalt- (Co) und Platin(Pt)-Katalysator.

12. Verfahren gemäß einem der vorstehenden Ansprüche, wobei der saure heterogene Katalysator von Schritt (e) ein Katalysator ist, der einen Aluminosilicat-Zeolith umfasst.

13. Verfahren gemäß Anspruch 12, wobei der saure heterogene Katalysator von Schritt (e) ZSM-5 umfasst.

14. Verfahren gemäß einem der vorstehenden Ansprüche, wobei der Katalysator von Schritt (e) ferner ein Übergangsmetall umfasst.

15. Verfahren gemäß Anspruch 14, wobei das Übergangsmetall Molybdän ist.

16. Verfahren gemäß einem der vorstehenden Ansprüche, wobei das Übergangsmetall in dem Katalysator von Schritt (f) Phosphinliganden aufweist.

17. Verfahren gemäß Anspruch 16, wobei der Phosphinligand des Übergangsmetalls (2,2'-[1,2-Phenylenbis[methylen[(1,1-dimethylethyl)phosphiniden]]]bis[pyridin] ist.

18. Verfahren gemäß einem der vorstehenden Ansprüche, wobei das Übergangsmetall in dem Katalysator von Schritt (f) Palladium ist.

19. Verfahren gemäß einem der vorstehenden Ansprüche, wobei die Säure bei Schritt (f) Essigsäure ist.

20. Verfahren gemäß einem der Ansprüche 1 bis 18, wobei die Säure bei Schritt (f) p-Toluolsulfonsäure ist.

**21.** Verfahren gemäß einem der vorstehenden Ansprüche, wobei die lineare Alkansäure, die 2 bis 5 Kohlenstoffatome umfasst, Essigsäure ist und die lineare Zwischenprodukt-Alkansäure, die 4 bis 7 Kohlenstoffatome umfasst, bei den Schritten (a) bis (c) Hexansäure oder ein Ester davon ist.

**22.** Verfahren gemäß Anspruch 21, wobei das lineare Alkanon, das 7 bis 28 Kohlenstoffatome umfasst, bei den Schritten (c) und (d) 6-Undecanon ist, das entsprechende sekundäre lineare Alkanol, das 7 bis 28 Kohlenstoffatome umfasst, bei den Schritten (d) und (e) 6-Undecanol ist, das lineare Alken, das 7 bis 28 Kohlenstoffatome umfasst, bei den Schritten (e) und (f) 5-Undecen ist und wobei die lineare Fettsäure, die 7 bis 28 Kohlenstoffatome umfasst, Dodecansäure ist.

## Revendications

**1.** Procédé de production d'acides gras linéaires comprenant 7 à 28 atomes de carbone ou des esters de ceux-ci, à partir d'éthanol et/ou d'un acide alcanoïque linéaire comprenant 2 à 5 atomes de carbone, le procédé comprenant

(a) la mise en contact d'éthanol et/ou de l'acide alcanoïque linéaire comprenant 2 à 5 atomes de carbone ou des sels de celui-ci avec au moins un microorganisme capable d'effectuer un allongement de chaîne de deux carbones pour produire en tant qu'intermédiaire un acide alcanoïque linéaire comprenant 4 à 7 atomes de carbone et/ou un sel de celui-ci et/ou un ester de celui-ci ;
(b) l'extraction de l'intermédiaire, son sel et/ou ester par (a) utilisation d'au moins un agent d'extraction, l'agent d'extraction comprenant au moins un oxyde d'alkyl-phosphine et éventuellement au moins un alcane comprenant au moins 12 atomes de carbone ; ou au moins une trialkylamine et au moins un alcane comprenant au moins 12 atomes de carbone ;
(c) la mise en contact de l'intermédiaire acide alcanoïque linéaire extrait comprenant 4 à 7 atomes de carbone, son sel et/ou ester de (b) avec au moins un catalyseur de cétonisation et éventuellement un acide alcanoïque supplémentaire comprenant 2 à 22 atomes de carbone dans des conditions de réaction adaptées pour la cétonisation chimique de l'intermédiaire acide alcanoïque linéaire comprenant 4 à 7 atomes de carbone, son sel et/ou ester en alcanone linéaire comprenant 7 à 28 atomes de carbone ;
(d) la mise en contact de l'alcanone linéaire comprenant 7 à 28 atomes de carbone de l'étape (c) avec au moins un catalyseur métallique d'hydrogénation pour l'hydrogénation catalytique de l'alcanone linéaire comprenant 7 à 28 atomes de carbone en alcanol linéaire secondaire correspondant comprenant 7 à 28 atomes de carbone ;
(e) la déshydratation de l'alcanol linéaire secondaire comprenant 7 à 28 atomes de carbone en présence d'un catalyseur hétérogène acide pour former un alcène linéaire correspondant comprenant 7 à 28 atomes de carbone ;
(f) l'hydroxy- ou alcoxy-carbonylation de l'alcène linéaire comprenant 7 à 28 atomes de carbone en acide linéaire gras comprenant 7 à 28 atomes de carbone ou un ester de celui-ci en présence de monoxyde de carbone, d'un acide et d'un catalyseur comprenant un métal de transition.

**2.** Procédé selon la revendication 1, dans lequel le catalyseur de cétonisation de (c) est un catalyseur à base d'oxyde métallique ou des mélanges de ceux-ci.

**3.** Procédé selon la revendication 2, dans lequel le catalyseur à base d'oxyde métallique ou des mélanges de ceux-ci est choisi dans le groupe constitué d'un catalyseur à base d'hétéropolyacide ($H_3PW_{12}O_{40}$), d'un catalyseur à base d'oxyde de titane ($TiO_2$), d'un catalyseur à base d'oxyde de cérium ($CeO_2$), d'un catalyseur à base d'oxyde mixte de zinc-chrome (Zn-Cr), d'un catalyseur à base d'oxyde de manganèse ($MnO_2$), d'un catalyseur à base d'oxyde de lanthane ($La_2O_3$), d'un catalyseur à base d'oxyde de magnésium (MgO), d'oxyde de fer (FeO, $FeO_2$, $Fe_2O_3$, $Fe_3O_4$, $Fe_4O_5$, $Fe_5O_6$, $Fe_5O_7$), d'un catalyseur à base d'oxyde mixte de silicium-aluminium (Si-Al) et d'un catalyseur à base d'oxyde de zirconium ($ZrO_2$).

**4.** Procédé selon l'une quelconque des revendications précédentes, dans lequel le catalyseur de cétonisation dans l'étape (c) est un catalyseur à base d'aérogel d'oxyde de zirconium.

**5.** Procédé selon l'une quelconque des revendications précédentes, dans lequel les conditions de réaction adaptées de l'étape (c) comprennent des températures de réaction de 150 °C à 350 °C.

**6.** Procédé selon l'une quelconque des revendications précédentes, dans lequel le microorganisme dans (a) est choisi dans le groupe constitué de *Clostridium carboxidivorans* et *Clostridium kluyveri.*

**7.** Procédé selon l'une quelconque des revendications précédentes, dans lequel l'oxyde d'alkyl-phosphine est choisi dans le groupe constitué de l'oxyde de trioctylphosphine, l'oxyde d'hexylphosphine, l'oxyde d'octylphosphine et des mélanges de ceux-ci et l'alcane est choisi dans le groupe constitué du pentadécane, de l'hexadécane, de l'heptadécane, de l'octadécane et du tétradécane.

**8.** Procédé selon l'une quelconque des revendications précédentes, dans lequel l'oxyde d'alkyl-phosphine est l'oxyde de trioctylphosphine (TOPO) et l'alcane est le tétradécane.

**9.** Procédé selon la revendication 8, dans lequel le rapport en poids du TOPO au tétradécane est compris entre 1:100 et 1:10.

**10.** Procédé selon l'une quelconque des revendications précédentes, dans lequel le pH du milieu aqueux dans (b) est maintenu entre 5,5 et 8.

**11.** Procédé selon l'une quelconque des revendications précédentes, dans lequel le catalyseur métallique d'hydrogénation de l'étape (d) est choisi dans le groupe constitué d'un catalyseur à base de ruthénium (Ru), d'un catalyseur à base de rhénium (Re), d'un catalyseur à base de nickel (Ni), d'un catalyseur à base de fer (Fe), de cobalt (Co) et de platine (Pt).

**12.** Procédé selon l'une quelconque des revendications précédentes, dans lequel le catalyseur hétérogène acide de l'étape (e) est un catalyseur comprenant une zéolite d'aluminosilicate.

**13.** Procédé selon la revendication 12, dans lequel le catalyseur hétérogène acide de l'étape (e) comprend ZSM-5.

**14.** Procédé selon l'une quelconque des revendications précédentes, dans lequel le catalyseur de l'étape (e) comprend en outre un métal de transition.

**15.** Procédé selon la revendication 14, dans lequel le métal de transition est le molybdène.

**16.** Procédé selon l'une quelconque des revendications précédentes, dans lequel le métal de transition dans le catalyseur de l'étape (f) comporte des ligands phosphine.

**17.** Procédé selon la revendication 16, dans lequel le ligand phosphine du métal de transition est la (2,2'-[1,2-phénylènebis[méthylène[(1,1-diméthyléthyl)phosphinidène]]]bis[pyridine].

**18.** Procédé selon l'une quelconque des revendications précédentes, dans lequel le métal de transition dans le catalyseur de l'étape (f) est le palladium.

**19.** Procédé selon l'une quelconque des revendications précédentes, dans lequel l'acide dans l'étape (f) est l'acide acétique.

**20.** Procédé selon l'une quelconque des revendications 1 à 18, dans lequel l'acide dans l'étape (f) est l'acide p-toluènesulfonique.

**21.** Procédé selon l'une quelconque des revendications précédentes, dans lequel l'acide alcanoïque linéaire comprenant 2 à 5 atomes de carbone est l'acide acétique et l'intermédiaire acide alcanoïque linéaire comprenant 4 à 7 atomes de carbone dans les étapes (a) à (c) est l'acide hexanoïque ou un ester de celui-ci.

**22.** Procédé selon la revendication 21, dans lequel l'alcanone linéaire comprenant 7 à 28 atomes de carbone dans les étapes (c) et (d) est la 6-undécanone, l'alcanol linéaire secondaire correspondant comprenant 7 à 28 atomes de carbone dans les étapes (d) et (e) est le 6-undécanol, l'alcène linéaire comprenant 7 à 28 atomes de carbone dans les étapes (e) et (f) est le 5-undécène et dans lequel l'acide gras linéaire comprenant 7 à 28 atomes de carbone est l'acide dodécanoïque.

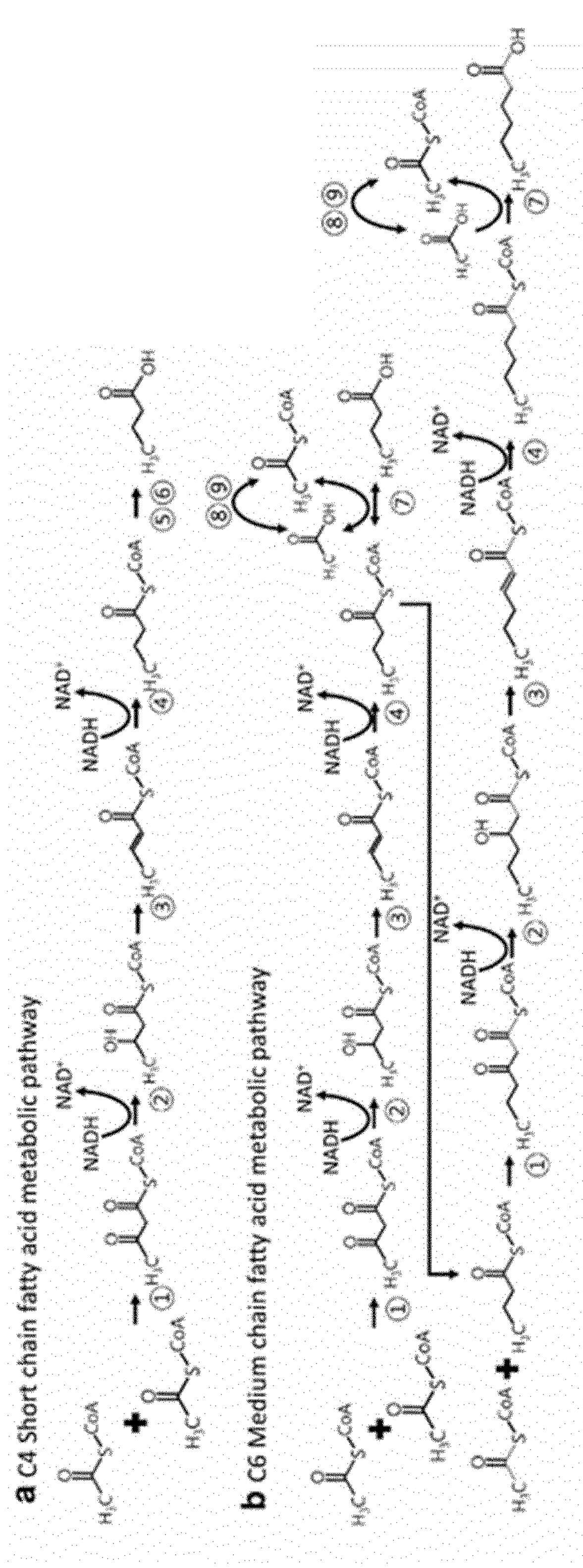


①; acetyl-CoA acetyltransferase, ②; 3-hydroxybutyryl-CoA dehydrogenase, ③; 3-hydroxybutyryl-CoA dehydratase, ④; butyryl-CoA dehydrogenase, ⑤; phosphotransbutyrylase, ⑥; butyrate kinase, ⑦; acetyl-CoA transferase, ⑧; phosphotransacetylase, ⑨; acetate kinase

FIGURE 1

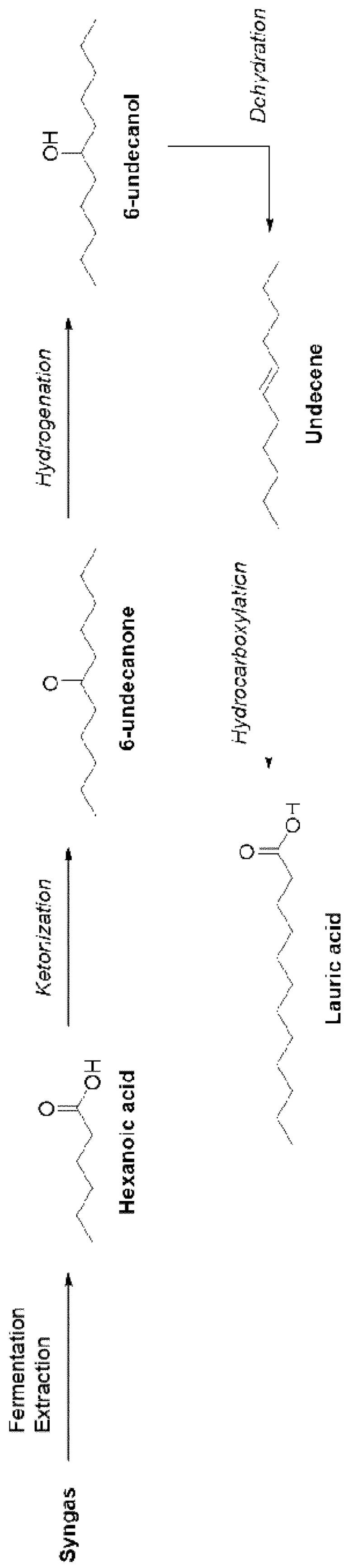


Syngas
Hexanoic acid
6-undecanone
6-undecanol
Undecene
Lauric acid

FIGURE 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4158668 A
- WO 2019158683 A
- EP 3538506 A
- US 20070275447 A
- US 20080057554 A
- WO 9800558 A
- WO 0068407 A
- US 6265618 B1


### Non-patent literature cited in the description


- **KIM BH et al.** *Appl Environ Microbiol.,* 1984, vol. 48 (4), 764-70
- **JEON et al.** *Biotechnol Biofuels,* 2016, vol. 9, 129
- **MORINAGA et al.** *J. Biotechnol.,* 1990, vol. 14, 187-194
- **SCHMIDT et al.** *Chem. Eng. Commun.,* 1986, vol. 45, 61-73
- **SAKAI et al.** *Biotechnol. Let.,* 2004, vol. 29, 1607-1612
- **WOO, Y.** *Ind. Eng. Chem. Res.,* 2017, vol. 56, 872-880
- **WANG, S.** *J. Phys. Chem. C,* 2017, vol. 121, 18030-18046
- **PHAM T. N.** *ACS Catal.,* 2013, vol. 3, 2456-2473
- **GLIRISKI, M. et al.** *Polish J. Chem.,* 2004, vol. 78, 299-302
- **OROZCO, L.M et al.** *ChemSusChem,* 2016, vol. 9 (17), 2430-2442
- **OROZCO, L.M et al.** *Green Chemistry,* 2017, vol. 19 (6), 1555-1569
- **LEE, Y. et al.** *Applied Catalysis A: General.,* 2015, vol. 506, 288-293
- **ALONSO, F.** *Tetrahedron,* 2008, vol. 64, 1847-52
- **GORGAS, N.** *Organometallics,* 2014, vol. 33 (23), 6905-6914
- **TARIQ SHAH M. et al.** *ACS Applied Materials & Interfaces,* 2015, vol. 7 (12), 6480-9
- **CHEN, J-X.** *Tetrahedron,* 2000, vol. 56, 2153-2166
- *Journal of Molecular Catalysis A: Chemical,* 2014, vol. 388-389, 116-122
- *ChemSusChem,* 2017, vol. 10 (11), 2527-2533
- *CHEMICAL ABSTRACTS,* 2093415-45-1